# EUROPEAN PATENT SPECIFICATION

(11) **EP 4 090 260 B1**
(45) Date of publication and mention of the grant of the patent: **11.09.2024**
(21) Application number: 21819975.0
(22) Date of filing: 29.11.2021
(51) Int. Cl.: A61B 17/072, A61B 90/92, A61B 90/98, A61B 17/00, A61B 90/00

(54) **SURGICAL SYSTEMS WITH DETACHABLE SHAFT RELOAD DETECTION**
CHIRURGISCHE SYSTEME MIT DETEKTION VON ABNEHMBAREN SCHÄFTEN ZUM NACHLADEN
SYSTÈMES CHIRURGICAUX AVEC DÉTECTION DE RECHARGEMENT D'ARBRE DÉTACHABLE

(30) Priority: 02.12.2020 US 202017109589; 02.12.2020 US 202017109636
(43) Date of publication of application: 23.11.2022
(73) Proprietor: Cilag GmbH International, 6300 Zug (CH)
(72) Inventor: SHELTON, IV, Frederick E., Cincinnati, Ohio 45242 (US); FIEBIG, Kevin M., Cincinnati, Ohio 45242 (US); BRADY, John E., Cincinnati, Ohio 45242 (US); MORGAN, Nicholas M., Cincinnati, Ohio 45242 (US)
(74) Representative: Carpmaels & Ransford LLP
(86) International application number: PCT/IB2021/061056
(87) International publication number: WO 2022/118165

(56) References cited:
- EP-A1- 2 944 276
- EP-A1- 3 047 806
- EP-A1- 3 165 178
- EP-A2- 1 943 976
- EP-A2- 3 064 145
- US-A1- 2020 297 439

## Description

### BACKGROUND

The present invention relates to surgical instruments and, in various arrangements, to surgical stapling and cutting instruments and staple cartridges for use therewith that are designed to staple and cut tissue.

EP 3 047 806 A1 discloses a powered surgical stapler which includes a housing, an endoscopic portion extending distally from the housing, and a loading unit configured to be removably attached to the endoscopic portion. The loading unit includes an end effector The stapler also includes a loading unit identification system including an identifier identifying the loading unit and being disposed thereon and an interrogator configured to interface with the identifier to obtain an identifying code uniquely associated with the loading unit.

### SUMMARY

The present invention provides a surgical system as recited in claim 1. Optional features are recited in the dependent claims.

### BRIEF DESCRIPTION OF THE DRAWINGS

Various features of the systems described herein, together with advantages thereof, may be understood in accordance with the following description taken in conjunction with the accompanying drawings as follows:
FIG. 1 illustrates an exemplary surgical device, according to at least one aspect of the present disclosure.
FIG. 2 illustrates a power-pack useable with the surgical device of FIG. 1, according to at least one aspect of the present disclosure.
FIG. 3 illustrates a housing and an adapter selectively coupleable with the housing, according to at least one aspect of the present disclosure.
FIG. 4 illustrates a handle assembly and a loading unit, according to at least one aspect of the present disclosure.
FIG. 5 illustrates a detailed view of the connection between the shaft assembly and the loading unit of FIG. 4, according to at least one aspect of the present disclosure.
FIG. 6 illustrates a graphical representation of capacitance detected by a control circuit over time, according to at least one aspect of the present disclosure.
FIG. 7 illustrates a distal end of a shaft assembly and a proximal end of a loading unit, according to at least one aspect of the present disclosure.
FIG. 8 illustrates a cross-sectional view of a loading unit, according to at least one aspect of the present disclosure.
FIG. 9 illustrates a cross-sectional view of a shaft assembly, according to at least one aspect of the present disclosure.
FIG. 10 illustrates the loading unit of FIG. 7 moving toward an aperture of the shaft assembly of FIG. 7 in an installation direction, according to at least one aspect of the present disclosure.
FIG. 11 illustrates the loading unit of FIG. 7 in an unlocked position with the shaft assembly of FIG. 7, according to at least one aspect of the present disclosure.
FIG. 12 illustrates the loading unit of FIG. 7 in a locked position with the shaft assembly of FIG. 7 according to at least one aspect of the present disclosure.
FIG. 13 illustrates a distal end of a shaft assembly and a proximal end of a loading unit, according to at least one aspect of the present disclosure.
FIG. 14 illustrates a cross-sectional view of the loading unit of FIG. 13, according to at least one aspect of the present disclosure.
FIG. 15 illustrates a cross-sectional view of the loading unit of FIG. 13 in an unlocked position with the shaft assembly of FIG. 13, according to at least one aspect of the present disclosure.
FIG. 16 illustrates a receptacle assembly and a resistor assembly, according to at least one aspect of the present disclosure.
FIG. 17 illustrates a circuit and a resistor assembly, according to at least one aspect of the present disclosure.
FIG. 18 illustrates a plurality of staple cartridges including resistor assemblies coupled thereto, according to at least one aspect of the present disclosure.
FIG. 19 illustrates a graphical representation of resistances determined by a control circuit of the resistor assemblies of FIG. 18, according to at least one aspect of the present disclosure.
FIG. 20 illustrates an exploded view of a mechanism for determining if a staple cartridge is properly seated in a cartridge channel, according to at least one aspect of the present disclosure.
FIG. 21 illustrates an unexploded view of the mechanism of FIG. 20, according to at least one aspect of the present disclosure.
FIG. 22 illustrates a shaft assembly including a J-shaped passage defined therein and a closed-end tunnel including a magnet therein, according to at least one aspect of the present disclosure.
FIG. 23 illustrates a detailed view of the J-shaped passage and the closed-end tunnel of FIG. 23, according to at least one aspect of the present disclosure.
FIG. 24 illustrates a magnet of an adapter positioned in a first passage portion of the J-shaped passage of FIG. 22, according to at least one aspect of the present disclosure.
FIG. 25 illustrates the magnet of FIG. 24 moved to a second passage portion of the J-shaped passage, according to at least one aspect of the present disclosure.
FIG. 26 illustrates the magnet of FIG. 24 moved to a third passage portion of the J-shaped passage, according to at least one aspect of the present disclosure.
FIG. 27 illustrates a J-shaped passage including a spring assembly positioned at a transition between the second passage portion and the third passage portion, according to at least one aspect of the present disclosure.
FIG. 28 illustrates the spring assembly of FIG. 27 in the compressed position and moving toward the expanded position to move a magnet of an adapter through the third passage portion, according to at least one aspect of the present disclosure.
FIG. 29 illustrates the spring assembly of FIG. 27 holding the magnet in the third passage portion, according to at least one aspect of the present disclosure.
FIG. 30 illustrates a graphical representation of outward resistive force by a magnet as a magnet moves through a J-shaped passage, according to at least one aspect of the present disclosure.
FIG. 31 illustrates a nozzle assembly and a handle assembly, according to at least one aspect of the present disclosure.
FIG. 32 illustrates a detailed view of a proximal end of the nozzle assembly of FIG. 31 and a distal end of the handle assembly of FIG. 31, according to at least one aspect of the present disclosure.
FIG. 33 illustrates a detailed view of the latch and contact arrangements of the nozzle assembly and handle assembly of FIG. 31, according to at least one aspect of the present disclosure.
FIG. 34 illustrates an alternative latch and switch arrangement of the nozzle assembly and handle assembly of FIG. 31, according to at least one aspect of the present disclosure.
FIG. 35 illustrates a graphical representation of a voltage detected by a control circuit of the latch and switch arrangement of FIG. 34 over time, according to at least one aspect of the present disclosure.
FIG. 36 illustrates a handle assembly, according to at least one aspect of the present disclosure.
FIG. 37 illustrates a top-down view of a handle assembly, according to at least one aspect of the present disclosure.
FIG. 38 illustrates a shaft assembly including a spring arrangement in an extended position, according to at least one aspect of the present disclosure.
FIG. 39 illustrates a shaft assembly including a spring arrangement in a compressed position according to at least one aspect of the present disclosure.
FIG. 40 illustrates a housing including a compressible material and an adapter selectively coupleable with the housing, according to at least one aspect of the present disclosure.
FIG. 41 illustrates a drive coupling assembly of an adapter and a compressible material in an uncompressed configuration, according to at least one aspect of the present disclosure.
FIG. 42 illustrates a drive coupling assembly of an adapter compressing a compressible material to a compressed configuration, according to at least one aspect of the present disclosure.

Corresponding reference characters indicate corresponding parts throughout the several views. The exemplifications set out herein illustrate certain systems, and such exemplifications are not to be construed as limiting the scope of the invention in any manner.

### DETAILED DESCRIPTION

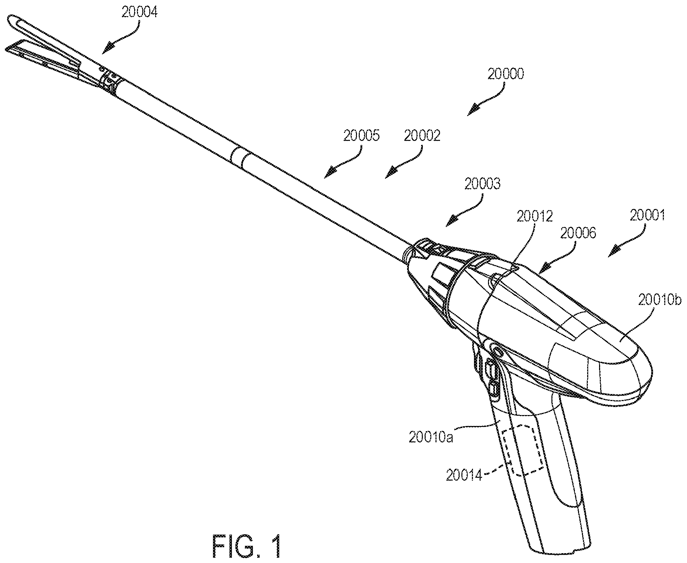

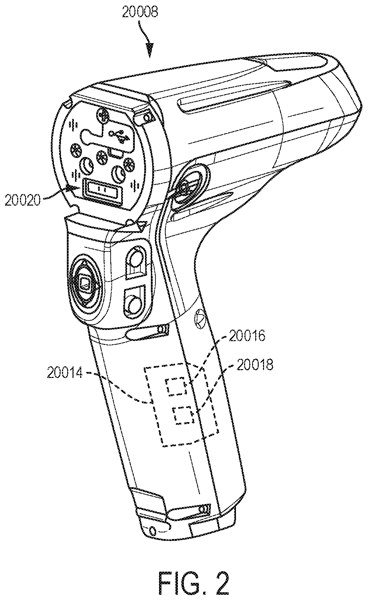

FIG. 1 depicts an exemplary surgical device 20000 that can include a handle assembly 20001 that can be selectively connectable with an adapter 20002, and, in turn, the adapter 20002 can be selectively connectable with end effectors or single use loading units ("SULU's") 20004. Alternatively, the adapter 20002 can be selectively connectable with multi-use use loading units ("MULU's"). The handle assembly 20001 can include an outer shell housing 20006 that is sized to selectively receive and substantially encase a power-pack 20008, illustrated in FIG. 2, therein that can drive various functions of the surgical device 20000, as explained below. The outer shell housing 20006 can include a distal half-section 2001 0a and a proximal half-section 2001 0b pivotably connected to distal half-section 2001 0a by a hinge 20012 located along an upper edge of distal half-section 20010a and proximal half-section 20010b. When joined, distal and proximal half-sections 2001 0a, 20010b define a shell cavity therein in which power-pack 20008 is selectively situated. The adapter 20002 can include an adapter housing 20003 that can mechanically and electrically couple to the outer shell housing 20006 and the power pack 20008, respectively, and a shaft assembly 20005 extending distally from the adapter housing 20003. In one aspect, the shaft assembly 20005 can mechanically and electrically couple to the end effector 20004.

In one aspect, the power pack 20008 can include a plurality of motors disposed therein for selectively driving various functions of the end effector 20004 when the surgical device is properly prepared for use. For example, rotation of motor shafts by respective motors function to drive shafts and/or gear components of the adapter 20002 in order to perform the various operations of surgical device 20000. In particular, motors of power-pack core assembly 20008 can drive shafts and/or gear components of adapter 20002 in order to selectively control functions of the end effector 20004. For example, motors can articulation the jaws of the end effector 20004 about an articulation joint, rotate the end effector 20004 about a longitudinal axis "X" extending through the adapter 20002, move a cartridge assembly of the end effector 20004 and an anvil assembly of end effector 20004 between an open position and a closed position to capture tissue therebetween, and/or to fire staples from within cartridge assembly of the end effector 20004, as examples. Alterntively, the end effector 20004 could include a radiofrequency (RF) or ultrasonic end effector where the motors can drive various functions of the RF or ultrasonic end effector. Additional functions of the motors are described in U.S. Patent No. 10,603,128.

The power pack 20008 can include a control system that can perform various operational functions of the surgical device 20000. For example, the control system can receive input signals from a user via input buttons or switches positioned on the outer shell housing 20006 to control various functions of the surgical device 20000, such as driving the motors, transmitting electrical communication signals to the end effector 20004, transmitting RF or ultrasonic drive signals to the end effector 20004, etc. The control system can include a control circuit 20014 in electrical communication with various electrical components disposed throughout the surgical device 20000. The control circuit 20014 can be in electrical communication with electrical components of the adapter 20002 and the SULU 20004 when the adapter 20002 is properly coupled to the outer shell housing 20006 and power pack 20008 and the end effector 20004 is properly coupled to the adapter 20002. For example, the power pack 20008 can include an electrical output portion 20020 and the adapter 20002 can include an electrical input portion. When the adapter 20002 is properly coupled to the outer shell housing 20006 and the power pack 20008, the electrical output portion 20020 and electrical input portion can be in electrical communication such that the control system can transmit electrical signals to the adapter 20002 and the end effector 20004. The control system can include a processor 20016 and a memory 20018 in communication with the processor. The memory 20018 can store instructions that can be executable by the processor 20016 to perform various operational functions of the surgical device 20000.

The control system can be in electrical communication with a display such that the control system can provide feedback to a user of the surgical device 20000. For example, the control system can provide visual indicators to the user about various functional parameters of the end effector 20004 coupled to the surgical device 20000. As another example, the display can provide visual feedback to the user about various interconnections between the surgical device 20000, such as the connection between the power pack 20008 and the housing assembly 20006 with the adapter 20002, or the adapter 20002 and the end effector 20004. The control system can further provide other forms of feedback to the user of the surgical device 20000 other than visual feedback, such as audible feedback, haptic feedback, or the like.

Currently, when a user attempts to connect the various components of the surgical device 20000 together, such as the outer shell housing 20006, the adapter 20002, the power pack 20008, the end effector 20004 as referenced above, the connections therebetween may be incomplete without the user knowing. In other instances, the connections therebetween may be complete, but the user has no way of knowing for sure whether or not this is the case. In such situations, attempting to operate the surgical device 20000 could raise safety concerns as the surgical device may fail to properly operate as intended due to the incomplete connection. For example, the motors of the power pack 20008 may be improperly coupled to the components of the adapter 20002 that are intended to be driven by the motors, or the electrical output portion 20020 may be improperly coupled to the electrical input portion of the adapter 20002. In other instances, the end effector 20004 may be improperly coupled to the adapter 20002 such that the adapter 20002 is unable to transmit electrical and mechanical signals from the power pack 20008 to the end effector 20004. It would therefore be desirable to ensure that components of a surgical device 20000 are properly connected and complete before utilizing the surgical device 20000 in a surgical procedure.

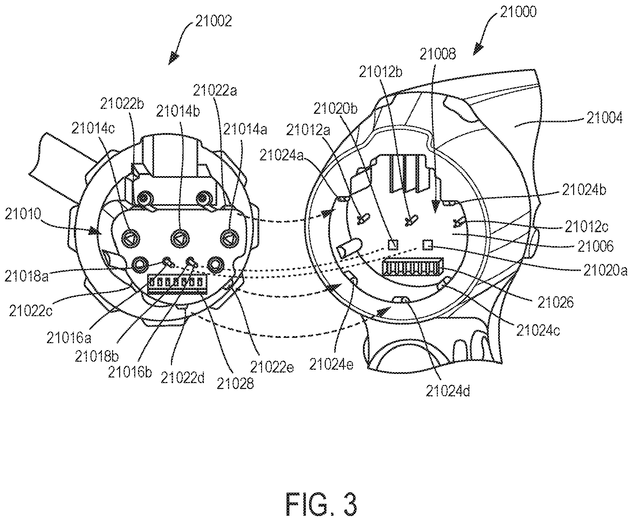

Referring now to FIG. 3, a housing assembly 21000 and adapter 21002 are provided, in accordance with at least one aspect of the present disclosure. The housing assembly 21000 can include an outer shell housing 21004 and a power pack 21006 disposed within the outer shell housing 21004. The outer shell housing 21004 and the power pack 21006 can be similar to outer shell housing 20006 and power pack 20008, respectively. The adapter 21002 can be similar to adapter 20002. The housing assembly 21000 can further include a recessed receiving area 21008 that is sized to receive a correspondingly shaped drive coupling assembly 21010 extending proximally from the adapter 21002. The housing assembly 21000 can further include a plurality of rotatable drive shafts 21012a, 21012b, 21012c extending from the receiving area 21008 of the housing assembly 21000. The power pack 21006 can include a plurality of motors operably coupled to the rotatable drive shafts 21012a, 21012b, 21012c that can drive the rotatable drive shafts 21012a, 21012b, 21012c.

The rotatable drive shafts 21012a, 21012b, 21012c can be sized such that, when the drive coupling assembly 21010 of the adapter 21002 is properly positioned within the receiving area 21008 of the housing assembly 21000, the drive shafts 21012a, 21012b, 21012c can be operably disposed within connecting sleeves 21014a, 21014b, 21014c of the drive coupling assembly 21010. More specifically, when the drive coupling assembly 21010 of the adapter 21002 is properly positioned within the receiving area 21008 of the housing assembly 21000, the first drive shaft 21012a can drivingly engage the first coupling sleeve 21014a, the second drive shaft 21012b can drivingly engage the second coupling sleeve 21014b, and the third drive shaft 21012c can drivingly engage the third coupling sleeve 21014c. When the drive shafts 21012a, 21012b, 21012c are in driving engagement with the coupling sleeves 21014a, 21014b, 21014c, rotation of the drive shafts 21012a, 21012b, 21012c can drive end effector functions of the surgical instrument. The end effector functions can be similar to those discussed elsewhere herein, such as moving jaws of an end effector between an open and closed position, translating a firing member proximally or distally within an end effector to cause stapling and severing of tissue positioned between the jaws of the end effector, or articulating the end effector about an articulation joint positioned proximal to the end effector, as examples. The drive shafts 21012a, 21012b, 21012c could also effect end effector functions of non-surgical stapling end effectors, such as RF or ultrasonic end effectors.

Continuing to refer to FIG. 3, the drive coupling assembly 21010 can further include a first shaft 21016a extending from a first channel 21018a defined in the drive coupling assembly 21010 and a second shaft 21016b extending from a second channel 21018b defined in the drive coupling assembly 21010. The first and second shafts 21016a, 21016b can be movably coupled to the drive coupling assembly 21010 such that the first and second shafts 21016a, 21016b can be movable between an extended position, illustrated in FIG. 3, wherein the shafts 21016a, 21016b are extending out of the channels 21018a, 21018b, and a depressed position, wherein the shafts 21016a, 21016b are at least partially depressed into the channels 21018a, 21018b. Each channel 21018a, 21018b can include a spring disposed therein such that the shafts 21016a, 21016b are 'pogo-stick' like shafts in that they are depressable toward the depressed position, but are biased toward the extended position when no force is applied thereto. As will be described in more detail below, the depressed positions of the shafts 21016a, 21016b can correspond to the adapter 21002 being completely and fully coupled to the housing assembly 21000.

The shafts 21016a, 21016b can be constructed of an electrically conductive material. In one aspect, the first and second shaft 21016a, 21016b can be in electrical communication with one another when both the first and second shaft 21016a, 21016b are in the depressed position, therefore signifying that the adapter 21002 is completely and fully coupled to the housing assembly 21000. In one example, an electrically conductive plate can be positioned at the distal end of both of the channels 21018a, 21018b such that, when the first and second shafts 21016a, 21016b are both in the depressed positions, a current can flow through the first shaft 21016a, through the conductive plate and then through the second shaft 21016b. In this way, a circuit can be formed between the first shaft 21016a and the second shaft 21016b when both the shafts 21016a, 21016b are in the depressed positions. While a conductive plate is described as being used to complete a circuit between the first and second shafts 21016a, 21016b when in the depressed positions, it should be understood that any suitable mechanism can be utilized to complete a circuit between the first and second shafts 21016a, 21016b when the first and second shafts 21016a, 21016b are in the depressed positions, such as a wire, a circuit board, or any suitable electrically conductive component positioned within the adapter 20002, as examples.

The housing assembly 21000 can further include a first contact 21020a and a second contact 21020b. The first and second contacts 21020a, 21020b are spaced such that, when the drive coupling assembly 21010 is properly positioned within the receiving area 21008, the first shaft 21016a can abut and be depressed by the first contact 21020a and the second shaft 21016b can abut and be depressed by the second contact 21020b. In one aspect, the contacts 21020a, 21020b can be comprised of an electrically conductive material and be in electrical communication with a control circuit positioned within the housing assembly 21000, such as control circuit 20014, as an example, such that an electrical potential can be generated between the two contacts 21020a, 21020b. When the drive coupling assembly 21010 is properly positioned within the receiving area 21008, the first contact 21020a can depress the first shaft 21016a to the depressed position and the second contact 21020b can depress the second shaft 21016b to the depressed position. When the shafts 21016a, 21016b are in the depressed positons, the control circuit can generate an electrical signal that can traverse through the first contact 21020a, the first shaft 21016a, the second shaft 21016b and the second contact 21020b, therefore signifying that the adapter 21002 is properly coupled to the housing assembly 21000. With such a system, when an electrical potential is generated at the contacts 21020a, 21020b and a circuit is unable to be completed, a user can know that the adapter 21002 is not properly coupled to the housing assembly 21000 and that appropriate action is required. The above-referenced system therefore provides a user with a mechanism for verifying if the adapter 21002 is properly coupled to the housing assembly 21000. The control circuit can provide feedback to a user, such as via a display, haptic feedback, or audible feedback, when the control circuit determines that the adapter 21002 is properly coupled to the housing 21000, as described above.

In one aspect, the drive coupling assembly 21010 can further include a plurality of flange features 21022a-e extending around the perimeter thereof. The flange features 21022a-e can be comprised of a substantially rigid material, such as a hard plastic, as an example. In addition, the housing assembly 21000 can include a plurality of flange features 21024a-e disposed about the receiving area 21008 that can correspond to the positions of the flange features 21022a-e of the drive coupling assembly 21010. The flange features 21024-e can be comprised of an elastomeric material such that the flange features 21024a-e can at least partially, elastically deform when a force is applied thereto, but can return to an undeformed state when the force is removed. In one aspect, a minimum threshold amount of force can be required to elastically deform the flange features 21024a-e to a deformed state.

In operation, when the drive coupling assembly 21010 of the adapter 21002 is moved toward the receiving area 21008 of housing assembly 21000, each of the plurality of flange features 21022a-e of the drive coupling assembly 21010 can abut the corresponding positioned flange features 21024a-e of the housing assembly 21010. Stated another way, flange feature 21022a can abut flange feature 21024a, flange feature 21022b can abut flange feature 21024b, flange feature 21022c can abut flange feature 21024c, flange feature 21022d can abut flange feature 21024d, and flange feature 21022e can abut flange feature 21024e. In order to properly seat the drive coupling assembly 21010 within the receiving area 21008 of the housing assembly 21000, once the flange features 21022a-e are abutting the corresponding positioned flange features 21024a-e, a user can apply a force to the adapter 21002 such that the flange features 21022a-e can cause the correspondingly positioned flange features 21024a-e to elastically deform, therefore allowing the flange features 21022a-e to pass the flange features 21024a-e.

In one aspect, the force applied by the user to the adapter 21002 can be large enough such that the flange features 21022a-e can apply a force to the correspondingly positioned flange features 21024a-e that meets or exceeds the minimum threshold amount of force to cause the flange features 21024a-e to elastically deform. Once the flange features 21022a-e pass the flange features 21024a-e, the flange features 21024a-e can return to their undeformed state, holding the flange features 21022a-d within the receiving area 21008, thereby holding the adapter 21002 to the housing assembly 21000. The flange features 21022a-e and flange features 21024a-e can be shaped such that, when the adapter 21002 is coupled to the housing assembly 21000, as described above, the flange features 21024a-e can releasably hold the flange features 21022a-e therein. In some examples, the flange features 21022a-e, 21024a-e can comprise ramp-like shapes, cylindrical shapes, or any suitable shape.

The use of the correspondingly positioned flange features 21022a-e, 21024a-e between the adapter 21002 and the housing assembly 21000 provides a mechanical means for a user to ensure that the adapter 21002 is properly seated and coupled with the housing assembly 21000 and that the adapter 21002 and housing assembly 21000 are properly rotatably aligned, owing to the positioning of the flange features 21022a-e, 21024a-e. In addition, the use of the correspondingly positioned flange features 21022a-e, 21024a-e between the adapter 21002 and the housing assembly 21000 can ensure that the adapter 21002 is maintained coupled to the housing assembly 21000 until a minimum threshold force is applied to the adapter 21002 to cause the flange features 21024a-e to elastically deform, thereby allowing the flange features 21022a-e to pass the flange features 21024a-e and exit the receiving area 21008.

In addition, the flange features 21022a-e, 21024a-e can be positioned to ensure that the first and second shafts 21016a, 21016b properly align with the contacts 21020a, 21020b, which, as described above, can be used as another level of security in ensuring that the adapter 21002 is both completely and properly coupled to the housing assembly 21000, thereby ensuring that operation of the housing assembly 21000, such as operation of the rotatable shafts 21012a-c, properly transmits forces and signals to the adapter 21002, such as to the coupling sleeves 21014a-c.

The housing assembly 21000 can further includes an electrical output connector 21026 coupled to the control circuit in the housing assembly 21000 and the adapter 21002 can include an electrical input connector 21028 sized to operably electrically couple to the electrical connector 21024 of the housing assembly 21000. In operation, when the electrical input connector 21026 is electrically, operably coupled to the electrical output connector 21028, the control circuit can transmit electrical signals, such as control signals or drive signals, such as RF or ultrasonic drive signals, from the housing assembly 21000 to the adapter 21002. In one aspect, a user can attempt to operate the surgical device utilizing the electrical connectors 21026, 21028 and the motors 21012a-c as a primary means of the determining if the housing assembly 21000 is properly coupled to the adapter 21002. A user can also use the above-described flange features 21022a-e, 21024a-e, shafts 21016a, 21016b and contacts 21020a, 21020b as a secondary means of ensuring that the electrical and mechanical connections between the housing assembly 21000 and the adapter 21002 are properly aligned and properly coupled to each other before operation of the surgical device.

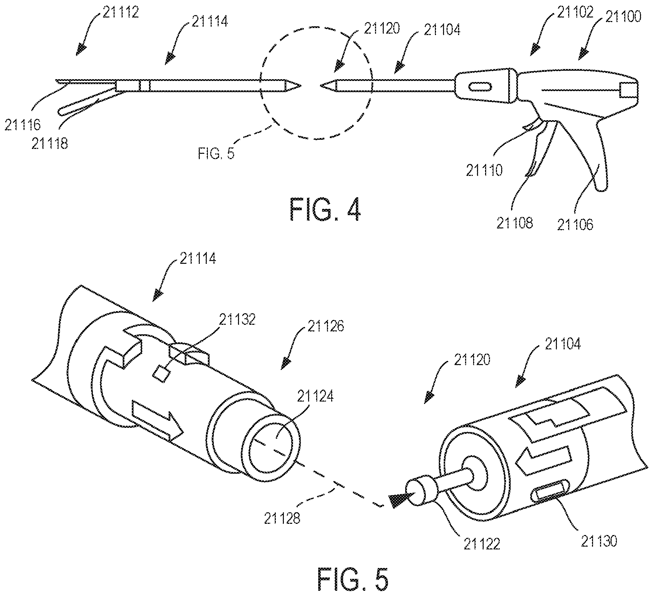

Referring now to FIG. 4, a mechanism for determining if a loading unit, such as a SULU or a MULU, is properly coupled and completely installed with a handle assembly is provided, according to at least one aspect of the present disclosure. A handle assembly 21100 can include a handle portion 21102 and a shaft assembly 21104 extending distally from the handle portion 21102. The handle assembly 21100 can be similar to handle assembly 20001 or housing assembly 21000. The shaft assembly 21104 could be similar to shaft assembly 20005. The handle portion 21102 can include a stationary handle 21106, a closure trigger 21108 and a firing trigger 21110. The closure trigger 21108 can be rotatable toward the stationary handle 21106 to transmit, for example, a closing motion to an end effector 21112 of a loading unit 21114 when the loading unit 21114 is properly attached to the shaft assembly 21104. The closing motion can cause a first jaw 21116 and a second jaw 21118 of the end effector 21112 to transition between an open configuration, wherein the first jaw 21116 and second jaw 21118 are spaced apart from one another, as shown in FIG. 4, and a closed configuration, wherein the first jaw 21116 and second jaw 21118 are spaced near each other to capture tissue therebetween. Similarly, the firing trigger 21110 can be rotatable toward the stationary handle 21106 to transmit, for example, a firing motion to the end effector 21112 when the loading unit 21114 properly attached to the shaft assembly 21104. The firing motion can cause staples to be deployed from the end effector 21112 into the tissue positioned between the first jaw 21116 and second jaw 21118, as well as cause a knife to sever the stapled tissue. Ahe first jaw 21116 can include an anvil and the second jaw 21118 can include a cartridge try with a staple cartridge removably positioned in the cartridge tray.

As is shown in FIG. 5, the distal end 21120 of the shaft assembly 21104 can include a drive shaft 21122 that can transmit actuation motions from the handle assembly 21100 to the loading unit 21114 when the loading unit 21114 is properly coupled and completely installed with the shaft assembly 21104. In one aspect, the drive shaft 21122 can be insertable into an aperture 21124 defined in the proximal end 21126 of the loading unit 21114. The loading unit 21114 can include a drive assembly sized to receive the drive shaft 21122 through the aperture 21124 such that, when the drive shaft 21122 is inserted into the aperture 21124, the drive assembly can operably couple to the drive shaft 21122. When coupled, actuation motions from the drive shaft 21122 can be transmitted to the drive assembly, allowing actuation motions from the handle assembly 21100 to be transferred to the end effector 21112 to effect end effector functions, such as closing motions, firing motions, articulation motions, etc., as described above. When the loading unit 21114 is properly coupled to the distal end 21120 of the shaft assembly 21104, the handle assembly 21100 can transmit electrical signals, such as communication or drive signals, to the loading unit 21114.

The loading unit 21114 can be properly coupled and completely installed with the shaft assembly 21104 by initially positioning the drive shaft 21122 into the aperture 21124. This can be accomplished, for example, by moving the aperture 21124 toward the drive shaft 21122 in an installation direction 21128 along an installation axis. In one aspect, the installation direction 21128 can be substantially parallel to a longitudinal axis defined through the shaft assembly 21104.

Once the drive shaft 21122 is inserted into the aperture 21124, the loading unit 21114 can be rotated relative to the shaft assembly 21104 about the longitudinal axis defined by the shaft assembly 21104. The loading unit 21114 can be rotatable relative to the shaft assembly 21104 between an unlocked position, where the loading unit 21114 can be moved away from the shaft assembly 21104 along the installation axis, and a locked position, wherein the loading unit 21114 is locked to the shaft assembly 21104, resulting in a loading unit 21114 that is properly coupled and completely installed with the shaft assembly 21104. Once the loading unit 21114 has rotated to the locked position, a locking mechanism can lock the loading unit 21114 to the shaft assembly 21104, thereby completely coupling and completely installing the loading unit with the shaft assembly. Once the loading unit 21114 is locked to the shaft assembly 21104, actuation motions and electrical signals from the handle assembly 21100 can be safety transmitted to the loading unit 21114 to effect end effector functions.

A user may desire to know if the loading unit 21114 is properly coupled to the shaft assembly 21104 prior to actuating the closure trigger 21108, actuating the firing trigger 21110, or attempting to transmit electrical signals to the loading unit 21114. For example, in instances where the loading unit 21114 wasn't completed rotated relative to the shaft assembly 21104 to the locked position and, therefore, wasn't completed locked into place, actuation motions or electrical signals from the handle assembly 21100, as an example, may not properly transfer to the loading unit 21114, and/or the loading unit 21114 may inadvertently decouple from the shaft assembly 21104 during the surgical procedure.

In addition, in various embodiments, the shaft assembly 21104 can comprise a first electrical contact and the loading unit 21114 can comprise a second electrical contact. When the loading unit 21114 is properly coupled to the shaft assembly 21104, the first and second electrical contact can be in electrical communication with other another such that electrical signals, such as RF or communication signals, can be transmitted between the shaft assembly 21104 and the loading unit 21114. These contacts can be in electrical communication with a control circuit that can utilize these contacts to determine if the loading unit 21114 is properly coupled to the shaft assembly 21104, such as by determining if a signal can be transmitted from the shaft assembly 21104 to the loading unit 21114. However, in some instances, these contacts may not properly detect that the loading unit 21114 is coupled to the shaft assembly 21104. It is therefore desirable to provide secondary means for determining if the loading unit 21114 is properly coupled to the shaft assembly 21104. It should be understood that the secondary means disclosed herein can be utilized as means for determining if any two components are coupled together, such as determining if a loading unit is properly coupled to an elongate shaft of a shaft assembly or determining if an adapter is properly coupled to a housing assembly, as examples.

In order to remedy the aforementioned problems, in various embodiments according to the present invention, the shaft assembly 21104 includes a first capacitor 21130 mounted to the distal end 21120 of the shaft assembly 21104. Similarly, the loading unit 21114 includes a second capacitor 21132 mounted to the proximal end 21126 of the loading unit 21114. In some embodiments, the first capacitor 21130 can be in electrical communication with a control circuit positioned in the handle assembly 21100, such as control circuit 20014, as an example. The capacitors 21130, 21132 can be positioned on the shaft assembly 21104 and the loading unit 21114, respectively, such that the control circuit can monitor a capacitance between the capacitors 21130, 21132 as the loading unit 21114 is coupled to the shaft assembly 21104, thereby allowing the control circuit to determine the location of the loading unit 21114 relative to the shaft assembly 21104, and therefore, determine if the loading unit 21114 is in the locked position.

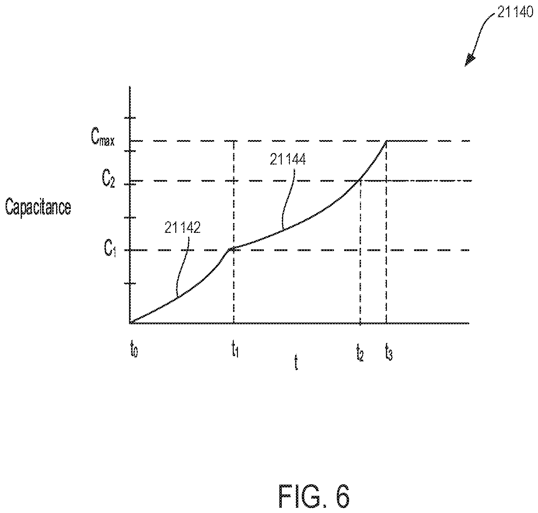

For example, referring now to FIG. 6, a graphical representation 21140 of capacitance detected by the control circuit over time is provided. In some embodiments, prior to the drive shaft 21122 being inserted into the aperture 21124 of the loading unit 21114 (t₀), the control circuit can detect no capacitance between the first capacitor 21130 and the second capacitor 21132. As the drive shaft 21122 is inserted into the aperture 21124, the control circuit can detect an increase 21142 in capacitance. For example, at t₁, a first capacitance C₁ can be detected by the control circuit between the first capacitor 21130 and the second capacitor 21132 as the loading unit 21114 is placed in the unlocked position relative to the shaft assembly 21104. In various embodiments, the first capacitance C₁ detected by the control circuit can be a predetermined capacitance level corresponding to the drive shaft 21122 being properly inserted into the aperture 21114 and being placed in the unlocked position. In various embodiments, the first capacitance level C₁ can correspond to the first capacitor 21130 and the second capacitor 21132 being angularly spaced apart from one another a first angle. In one aspect, when the control circuit detects a capacitance that is less than the first capacitance C₁, the control circuit can provide feedback, such as through a display coupled to the control circuit, haptic feedback, audible feedback, etc., indicating that the drive shaft 21122 isn't properly inserted into the aperture 21114, indicating to a user that a corrective action is required prior to rotating the loading unit 21114 to the locked position.

As described above, to completely couple the loading unit 21114 to the shaft assembly 21104, the loading unit 21114 can be rotated relative to the shaft assembly 21104 to the locked position to lock and completely couple and install the loading unit 21114 to the shaft assembly 21104. As illustrated in FIG. 6, as the drive shaft 21122 is rotated relative to the shaft assembly 21104, the control circuit can detect an increase 21144 in capacitance between the first capacitor 21130 and the second capacitor 21132 as the second capacitor 21132 slides relative to the first capacitor 21130. For example, at t₂, a second capacitance C₂ can be detected by the control circuit between the first capacitor 21130 and the second capacitor 21132. In various embodiments, the second capacitance C₂ detected by the control circuit can be a capacitance level that is less than a predetermined maximum capacitance Cₘₐₓ, where Cₘₐₓ corresponds to the loading unit 21114 not being completely rotated relative to the shaft assembly 211104 to the locked position, therefore signifying that the loading unit 21114 is not properly coupled to the shaft assembly 21104. At t₂, as the control circuit detects a capacitance level C₂ that is less than the predetermined maximum capacitance Cₘₐₓ, the control circuit can alert a user, via the display, haptic feedback, audible feedback, etc., that the loading unit 21114 is not properly coupled to the shaft assembly 21104 and that further rotation toward the locked position is required.

As further illustrated in FIG. 6, as the loading unit 21114 continues to rotate relative to the shaft assembly 21104, the control circuit can continue to detect an increase 21144 in capacitance between the first capacitor 21130 and the second capacitor 21132 as the second capacitor 21132 slides relative to the first capacitor 21130. For example, at t₃, a capacitance detected by the control circuit between the first capacitor 21130 and the second capacitor 21132 can meet or exceed the predetermined maximum capacitance Cₘₐₓ. As the control circuit detects a capacitance level that is substantially equal to or greater than the predetermined maximum capacitance Cₘₐₓ, the control circuit can alert a user, via the display, haptic feedback, audible feedback, etc., that the loading unit 21114 is properly coupled to the shaft assembly 21104 and that no further rotation is required.

In addition to the above-described capacitance assembly, the loading unit 21114 can be provided with a dielectric thereon that is able to be read and interpreted by the control circuit. In one aspect, the control circuit can interpret the dielectric to determine a type of loading unit 21114 that is coupled to the shaft assembly 21104. The control circuit can interpret the dielectric to determine any number of parameters associated with the loading unit 21114, such as the length of the loading unit, the type of loading unit (RF, ultrasonic, stapling, etc.), the height of the staples positioned in the staple cartridge of a stapling end effector, the orientation of the staples in the staple cartridge, the length of the staples, the length of the anvil coupled to the loading unit 21114, as examples.

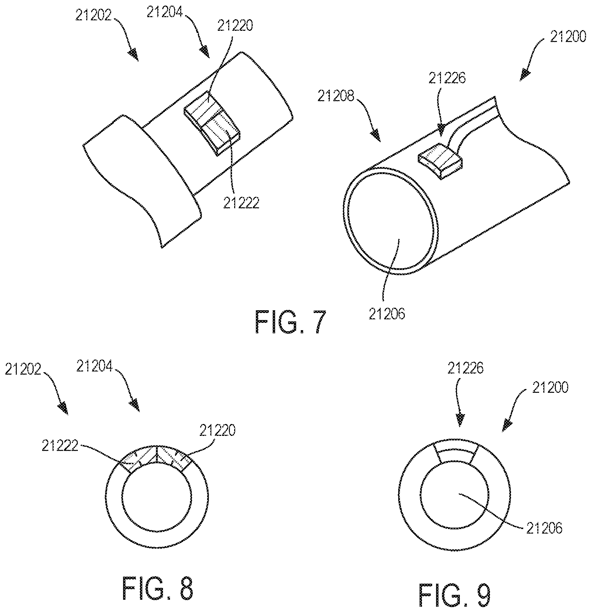

Referring now to FIGS. 7-9, another mechanism for determining if a loading unit, such as a SULU or a MULU, is properly coupled and completely installed with a handle assembly is provided, according to the present invention. A shaft assembly 21200 and a loading unit 21202 are provided. The shaft assembly 21200 can be similar to shaft assembly 20005 and/or shaft assembly 21104 and the loading unit 21202 can be similar to loading unit 21114 and/or loading unit 20004. The shaft assembly 21200 can extend from a housing assembly, such as the housing assemblies 20001, 21000, 21100, as examples, and can facilitate transmission of actuation motions from the housing assembly to the loading unit 21202 when the loading unit 21202 is properly coupled and completely installed therewith.

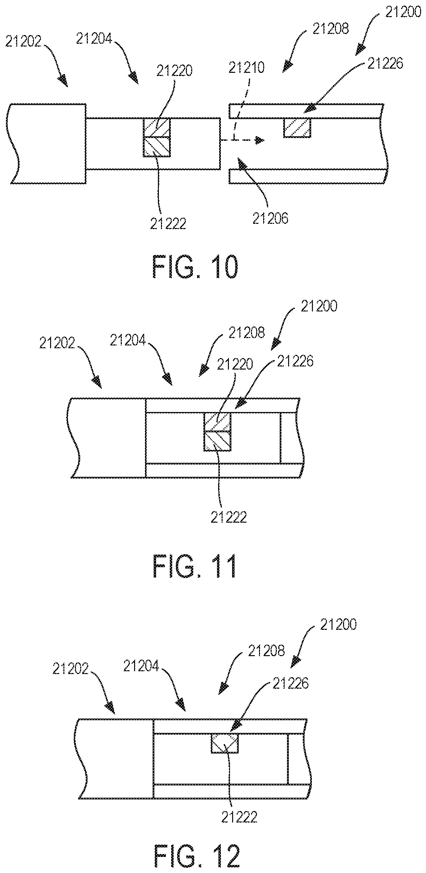

The loading unit 21202 can be properly coupled and completely installed with the shaft assembly 21200 by initially positioning a proximal end 21204 of the loading unit 21202 into an aperture 21206 defined at a distal end 21208 of the shaft assembly 21200. This can be accomplished, as an example, referring to FIG. 10, by moving the proximal end 21204 of the loading unit 21202 toward the aperture 21206 in an installation direction 21210 along an installation axis. The installation direction 21128 can be substantially parallel to a longitudinal axis defined through the shaft assembly 21200. Once the proximal end 21204 of the loading unit 21202 is inserted into the aperture 21206, the loading unit 21202 can be rotated relative to the shaft assembly 21200 about the longitudinal axis defined by the shaft assembly 21200. The loading unit 21202 can be rotatable relative to the shaft assembly 21200 between an unlocked position, where the loading unit 21202 can be moved away from the shaft assembly 21200 along the installation axis, and a locked position, wherein the loading unit 21202 is locked to the shaft assembly 21200. Once the loading unit 21202 has rotated to the locked position, a locking mechanism can lock the loading unit 21200 to the shaft assembly 21200, thereby completely coupling and completely installing the loading unit 21202 with the shaft assembly 21200. Once the loading unit 21202 is locked to the shaft assembly 21200, actuation motions and electrical signals from the handle assembly can be safety transmitted from the shaft assembly 21200 to the loading unit 21202 to effect end effector functions.

A user may desire to know if the loading unit 21202 is properly coupled to the shaft assembly 21200 prior to transmitting actuation motions and electrical signals to the loading unit 21202 through the shaft assembly 21200. For example, in instances where the loading unit 21202 wasn't completed rotated relative to the shaft assembly 21200 to the locked position and, therefore, wasn't completed locked into place, actuation motions and electrical signals from the handle assembly may not properly transfer to the loading unit 21202, or the loading unit 21202 may inadvertently decouple from the shaft assembly 21200 during the surgical procedure.

In various embodiments according to the present invention, the loading unit 21202 can include a first magnet 21220 and a second magnet 21222. The first magnet 21220 can include a first polarity and the second magnet 21222 can include a second polarity that is different that the first polarity. In one example embodiment, the second polarity can be opposite of the first polarity. The first magnet 21220 and the second magnet 21222 can be coupled to the proximal end 21204 of the loading unit 21202. In addition, the shaft assembly 21200 includes a sensor assembly 21226 which can be coupled to the distal end 21208 of the shaft assembly 21200. In some embodiments, the sensor assembly 21226 can be in electrical communication with a control circuit positioned in the handle assembly, such as control circuit 20014, as an example. In various embodiments, the sensor assembly 21226 can comprise a Hall-effect sensor that can sense a polarity of the first magnet 21220 and the second magnet 21222 to determine a position of the loading unit 21202 relative to the shaft assembly 21200 when the loading unit 21202 is coupled to the shaft assembly 21200. In various embodiments, referring to FIGS. 8 and 9, the magnets 21222, 21220 and the sensor assembly 21226 can be integral to the loading unit 21202 and the shaft assembly 21200.

In one aspect, when the loading unit 21202 is coupled to the shaft assembly 21200, the sensor assembly 21226 can sense a polarity of the first magnet 21220 and the second magnet 21222 and transmit a signal to the control circuit indicative of the sensed polarity. The control circuit can interpret the detected polarity to determine a position of the loading unit 21202 relative to the shaft assembly 21200. In some embodiments, when the loading unit 21220 is initially moved to the unlocked position along the installation axis 21210, as is shown in FIGS. 10 and 11, the sensor assembly 21226 can detect first polarity of the first magnet 21220. The control circuit can interpret this first polarity and determine that the first magnet 21220 is positioned at least substantially adjacent to the sensor assembly 21226, indicating that the loading unit 21202 is in the unlocked position and not yet completely installed or coupled to the shaft assembly 21200. In various embodiments, the control circuit can provide feedback, such as visual through a display, audible, or haptic, as examples, of the control circuit determining that the loading unit 21202 is in the unlocked position.

As discussed above, in the unlocked position, the loading unit 21202 can be rotated relative to the shaft assembly 21200 about a longitudinal axis defined by the shaft assembly 21200. As the loading unit 21202 rotates toward the locked position, the first magnet 21220 can move away from the sensor assembly 21226 and the second magnet 21222 can move toward the sensor assembly 21226. The control circuit can, through the sensor assembly 21226, determine that the second magnet 21222 is moving toward the sensor assembly 21226 by sensing the polarity shift of the first magnet 21220 to the second magnet 21222, thereby allowing the control circuit to monitor the rotation of the loading unit 21202. The second magnet 21222 can continue to be rotated toward the sensor assembly 21226 until the second magnet 21226 is adjacently positioned to the sensor assembly 21226, as is shown in FIG. 12. In various embodiments, the second magnet 21222 being adjacently positioned to the sensor assembly 212260 can be indicative of the loading unit 21202 being in the locked and fully coupled orientation with the shaft assembly 21200. Once the second magnet 21222 reaches the adjacent relationship with the sensor assembly 21226, thereby indicating that the loading unit 21202 is in the locked and fully coupled orientation with the shaft assembly 21200, the control circuit can provide feedback to the user, via visual, audible, haptic, or the like, indicating that the loading unit 21202 is properly coupled to the shaft assembly 21200, and is therefore safe to use.

In various aspects, the control circuit can determine that the loading unit 21202 is in the locked position by monitoring the sensor assembly 21226 and comparing a sensed value of the sensor assembly 21226 to a predetermined threshold. As one example, when the control circuit interrogates the sensor assembly 21226 and determines that the value sensed by the sensor assembly 21226 has reached or exceeded the predetermined threshold, the control circuit can conclude that the loading unit 21202 is in the locked position. As another example, when the control circuit interrogates the sensor assembly 21226 and determines that the value sensed by the sensor assembly 21226 has not yet reached the predetermined threshold, the control circuit can conclude that the loading unit 21202 is not in the locked position and further rotation is required.

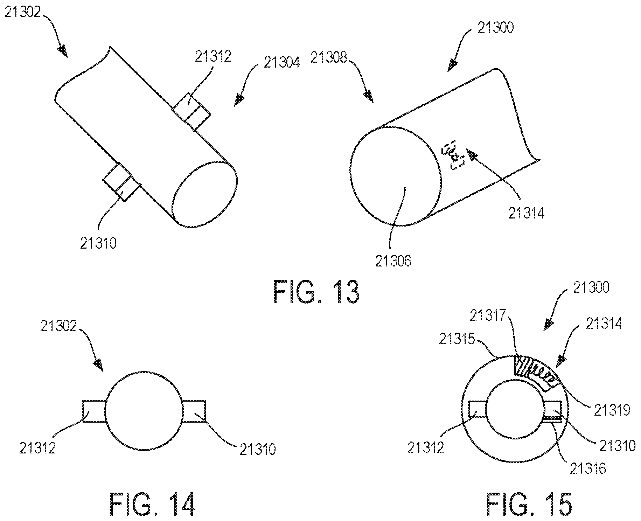

Referring now to FIG. 13, a mechanism for ensuring that a loading unit, such as a SULU or a MULU, is properly coupled to a shaft assembly is provided, according to at least one aspect of the present disclosure. A shaft assembly 21300 and a loading unit 21302 are provided. The shaft assembly 21300 can be similar to shaft assembly 21200, shaft assembly 20005 and/or shaft assembly 21104 and the loading unit 21302 can be similar to loading unit loading unit 21202, loading unit 21114, and/or loading unit 20004. The shaft assembly 21300 can extend from a housing assembly, such as the housing assemblies 20001, 21000, 21100, as examples, and can facilitate transmission of actuation motions and electrical signals from the handle assembly to the loading unit 21302 when the loading unit 21302 is properly coupled and completely installed therewith.

The loading unit 21302 can be properly coupled and completely installed with the shaft assembly 21300 by initially positioning a proximal end 21304 of the loading unit 21302 into an aperture 21306 defined at a distal end 21308 of the shaft assembly 21300. This can be accomplished, as an example, by moving the proximal end 21304 of the loading unit 21302 toward the aperture 21306 in an installation direction, similar to installation direction 21128 or installation direction 21210, along an installation axis. The installation direction can be substantially parallel to a longitudinal axis defined through the shaft assembly 21300.

Once the proximal end 21304 of the loading unit 21302 is inserted into the aperture 21306, the loading unit 21302 can be rotated relative to the shaft assembly 21300 about the longitudinal axis defined by the shaft assembly 21300. The loading unit 21302 can be rotatable relative to the shaft assembly 21300 between an unlocked position, where the loading unit 21302 can be moved away from the shaft assembly 21300 along the installation axis, and a locked position, wherein the loading unit 21302 is locked to the shaft assembly 21300. Once the loading unit 21302 has rotated to the locked position, a locking mechanism can lock the loading unit 21300 to the shaft assembly 21300, thereby completely coupling and completely installing the loading unit 21302 with the shaft assembly 21300. Once the loading unit 21302 is locked to the shaft assembly 21300, actuation motions and electrical signals from the handle assembly can be safety transmitted to the loading unit 21302 through the shaft assembly 21300 to effect end effector functions.

In one aspect, a user may desire to know if the loading unit 21302 is properly coupled to the shaft assembly 21300 prior to transmitting actuation motions and electrical signals to the loading unit 21302. For example, in instances where the loading unit 21302 wasn't completed rotated relative to the shaft assembly 21300 to the locked position and, therefore, wasn't completed locked into place, actuation motions and electrical signals from the handle assembly may not properly transfer to the loading unit 21302, or the loading unit 21302 may inadvertently decouple from the shaft assembly 21300 during the surgical procedure.

The loading unit 21302 can include a first lug or flange 21310 extending a first lateral direction from the proximal end 21304 of the loading unit 21302 and a second lug or flange 21312 extending from a second lateral direction from the proximal end 21304 of the loading unit 21302. The first lateral direction can be opposite the first lateral direction, as is shown in FIGS. 13-15. The first lateral direction can be perpendicular to the second lateral direction. Any suitable angle can be defined between the first lateral direction and the second lateral direction such that the first lateral direction is different than the first lateral direction. While two lugs 21310, 21312 are shown and described, it should be understood that fewer or more than two lugs can be utilized without diverting from the scope of the disclosure that will be described below.

In addition, the shaft assembly 21300 can include a spring assembly 21314 extending from an inner wall 21315 of the shaft assembly 21300. The spring assembly 21314 can include a base 21317 mounted to the inner wall 21315 and a spring 21319 extending from the base, as shown best in FIG. 15. In one aspect, the spring 21319 can comprise a linear spring or a torsional spring, as examples, such that the spring assembly 21314 is able to provide a biasing force against one of the first lug 21310 or second lug 21312 when a force is applied to the spring assembly 21314 by the same, as will be described in more detail below.

Similar to other loading units and shaft assemblies disclosed herein, to completely couple the loading unit 21302 to the shaft assembly 21300, the loading unit 21302 can first be brought into an unlocked position with the shaft assembly 21300, as described above. As the loading unit 21302 is moved toward the unlocked position, the first lug 21310 and the second lug 21312 can move through the aperture 21306 and be positioned within the shaft assembly 21300 such that the first lug 21310 and the second lug 21312 are radially aligned with the spring assembly 21314, as is shown in FIG. 15. To bring the loading unit 21302 to the locked position, as was described above, the loading unit 21302 can be rotated relative to the shaft assembly 21300 toward the locked position. Once the loading unit 21302 has rotated to the locked position, a locking mechanism can lock the loading unit 21300 to the shaft assembly 21300, as referenced above, thereby completely coupling and completely installing the loading unit 21302 with the shaft assembly 21300.

The shaft assembly 21300 could include a switch, such as an on-off switch, that can be in electrical communication with a control circuit in the housing assembly, such as control circuit 20014. One of the lugs can abut the on-off switch when the loading unit 21302 reaches the locked position. The control circuit can identify that the on-off switch has been actuated and provide feedback, such as visual with a display, audible, or haptic, as examples, to a user indicating that the loading unit 21302 has been placed in the locked position.

In one aspect, as the loading unit 21302 is rotated toward the locked position, the first lug 21310 can abut the spring 21319 of the spring assembly 21314. The spring 21319 can resist rotation of the first lug 21310 as the loading unit 21310 moves toward the locked position. In order to completely couple the loading unit 21302 with the shaft assembly 21300, the loading unit 21302 can be rotated toward the locked position with such a force so as the first lug 21310 can impart a sufficient amount of force to overcome the spring bias of the spring 21319 and enter into the locked position. In instances where the loading unit 21302 is only partially rotated to the locked position, the spring assembly 21314 can bias the loading unit 21302 toward the unlocked position by applying a resistive force to the first lug 21310. Thus, the spring assembly 21314 is configured to give haptic feedback to a user attempting to rotate the loading unit 21302 toward the locked position in the form of the resistive force. In the locked position, the user no longer feels the resistive force. Additionally, in certain instances, entering the locked position yields audible feedback in the forming of a clicking sound, for example.

As described above, the spring assembly 21314 provides a mechanism to ensure that the loading unit 21302 is completely placed in the locked position prior to the shaft assembly 21300 and loading unit 21302 being used in a surgical procedure. If the loading unit 21302 is not rotated to the completely to the locked position, the spring 21319 can bias the loading unit 21302 to the unlocked position, allowing a user to identify that the loading unit 21302 has not been properly attached and that corrective action is required. The spring assembly 21314 prevents the loading unit 21302 from entering the locked configuration unless a threshold amount of force is applied to the spring assembly 21314 by the first flange 21310 so as to overcome the spring bias of the spring assembly 21314.

The shaft assembly 21300 can further include a stop member 21316 extending from the inner wall 21315 of the shaft assembly 21300. The stop member 21316 can be sized and positioned such that, should the loading unit 21302 be rotated to the unlocked position by the spring 21314, the stop member 21316 both prevents the loading unit from rotating beyond the unlocked position, as well as prevents the spring bias of the spring 21319 from forcing the loading unit 21302 out of the aperture 21306 of the shaft assembly 21300. The stop member 21316 can be sized and positioned such that, as the spring 21319 forces the loading unit to the unlocked position, the stop member 21316 can abut one of the lugs 21310, 21312 in the unlocked position to prevent the spring bias force of the spring 21319 from forcing the loading unit 21302 out of the aperture 21306. The stop member 21316 can therefore require that the loading unit 21302 be removed from the aperture 21306 along the linear, installation axis. The stop member 21316 can be positioned slightly offset the unlocked position such that, in the unlocked position, the loading unit 21302 can be rotated slightly toward the locked position to disengage the stop member 21316 from one of the lugs 21310, 21312 and then moved along the installation axis to remove the loading unit 21302 from the aperture. The above described stop member 21316 can be utilized in any systems described herein that require one component to rotate relative to another component to move between a locked and unlocked position. While one stop member 21316 was described, it should be understood that more than one stop member 21316 can be used. For example, there can be a 1:1 ratio of lugs to stop members 21316.

Alternatively, the shaft assembly 21300 can further include a second spring assembly positioned on an opposite side of the shaft assembly 21300 such that the first spring assembly 21314 can resist rotation of the first flange 21310 and the second spring assembly can resist rotation of the second flange 21312. The use of a second spring assembly can further increase the threshold force required for the loading unit 21302 to enter the locked position. Various other systems are envisioned where the loading unit 21302 includes a 1:1 ration of flanges to spring assemblies.

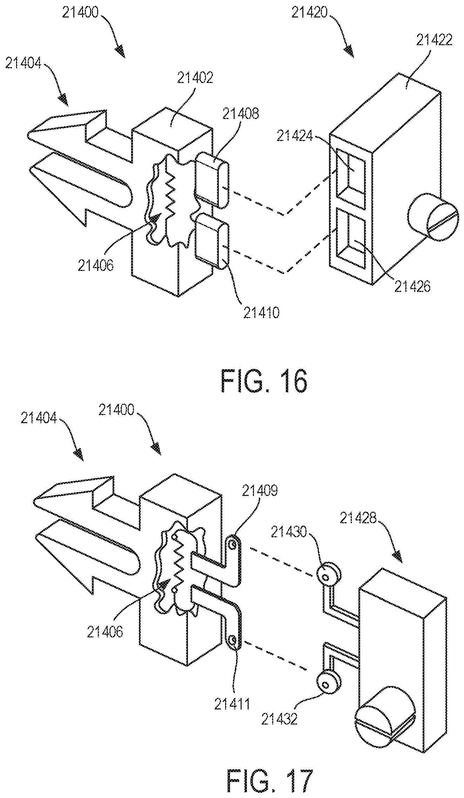

Referring now to FIGS. 16 and 17, a mechanism for determining if a staple cartridge is properly seated in a cartridge channel of an end effector and a type of staple cartridge that is seated in the cartridge channel is provided, according to at least one aspect of the present disclosure. A staple cartridge can include a resistor assembly 21400 operably coupled thereto. In one aspect, the resistor assembly 21400 can include a housing 21402, an attachment feature 21404 extending from the housing 21402 to removably attach the resistor assembly 21400 to the cartridge, a circuit 21406 disposed within the housing 21402, a first arm 21408 and a second arm 21410. The first arm 21408 can include a first contact arm 21409 disposed therein and the second arm 21410 can include a second contact arm 21411 disposed therein. Alternatively, the first contact arm 21409 and the second contact arm 21411 extent from the housing 21408 and are not disposed within the first arm 21408 and the second arm 21410. Stated another way, the resistor assembly 21400, does not employ the first arm 21408 and the second arm 21410.

The circuit 21406 can be tuned with a predetermined resistance value that corresponds to a type of cartridge to which the resistor assembly 21400 is coupled thereto. In one system, a circuit 21406 with a resistance R1 can correspond to a staple cartridge that includes staples with a staple height H1. In another system, a circuit 21406 with a resistance R2 can correspond to a staple cartridge that includes staples with a staple height H2, where H2 is different than H1. In another system, a circuit 21406 with a resistance R3 can correspond to a staple cartridge that includes a cartridge length of L3. In another system, a circuit 21406 with a resistance R4 can correspond to a staple cartridge that includes a cartridge length of L4, where L4 is different than L3. Any number of resistance values of the circuit 21406 can correspond to any number of staple cartridge parameters, such as staple size, staple height, cartridge length, or the like. A unique resistance value of the circuit 21406 can correspond to more than one parameter of the staple cartridge. In one system, a circuit with a resistance of R1 can correspond to a staple cartridge that includes staples having a staple height H1 and a cartridge with a length L1, as an example. Various other systems are envisioned where the resistor assembly 21400 can be coupled to cartridges other than staple cartridges, such as RF cartridges, where the resistance value of the circuit 21406 can correspond to various parameters associated with the cartridges.

An end effector of a surgical instrument can include a cartridge channel that is sized to receive a staple cartridge therein. In some situations, it would be desirable to ensure that the staple cartridge is properly seated in the cartridge channel prior to the staple cartridge being utilized in a surgical procedure. The cartridge channel can be provided with a receptacle assembly 21420 that includes housing 21422, a first window 21424, a second window 21426, a circuit 21428, a first contact arm 21430 extending from the circuit 21428 and positioned in the first window 21424 and a second contact arm 21432 extending from the circuit 21428 and positioned in the second window 21426. Various other systems are envisioned where the receptacle assembly 21420 does not include the housing 21420, the first window 21424, or the second window 21426 and instead merely includes the circuit 21428, the first contact arm 21430 and the second contact arm 21432.

In certain instances, the housing 21422, or at least a portion thereof, is comprised of an insulative material such as a polymer, more specifically a polyimide, polyester, fluorocarbon, or any polymeric material, or any combinations thereof. In certain instances, the contact arms 21430, 21432 are comprised of an electrically conductive materials such as, for example, a metal.

In one aspect, the circuit 21428 can be in electrical communication with a control circuit positioned within a housing assembly, such as control circuit 20014, as an example, that is operably coupled with the cartridge channel of the end effector. The first window 41424 and second window 21426 are sized such that, when a staple cartridge including a resistor assembly 21400 is properly seated within the cartridge channel, the first arm 21408 of the resistor assembly 21400 is inserted into the first window 21424 and the second arm 21410 is inserted into the second window 21426. When the first arm 21408 is positioned in the first window 21424 and the second arm 21410 is positioned in the second window, the circuit 21428 can electrically communicate with the circuit 21406. More specifically, when the first arm 21408 is positioned in the first window 21424, the first contact arm 21409 can electrically communicate with the first contact arm 21430 and the second contact arm 21411 can electrically communicate with the second contact arm 21432, thereby completing the circuit from the circuit 21428 to the circuit 21406. Alternatively, when a staple cartridge including a resistor assembly 21400 is properly seated within the cartridge channel, a user can determine that the staple cartridge is properly positioned in the cartridge channel if the first contact arm 21430 and the second contact arm 21432 are able to electrically communicate with the first contact arm 21409 and the second contact arm 21411, as will be discussed in more detail below.

In one aspect, when the circuit 21428 is in operable electrical communication with the circuit 21406, the control circuit of the housing assembly can transmit an electrical signal through the circuit 21428 to the circuit 21406 of the resistor assembly 21400, therefore verifying that the staple cartridge is properly positioned in the cartridge channel. In a scenario where a user attempts to verify if the staple cartridge is properly positioned in the cartridge channel and a complete circuit is not able to be made, as described above, a user is able to determine that the staple cartridge is not properly positioned in the cartridge channel and that appropriate action is required.

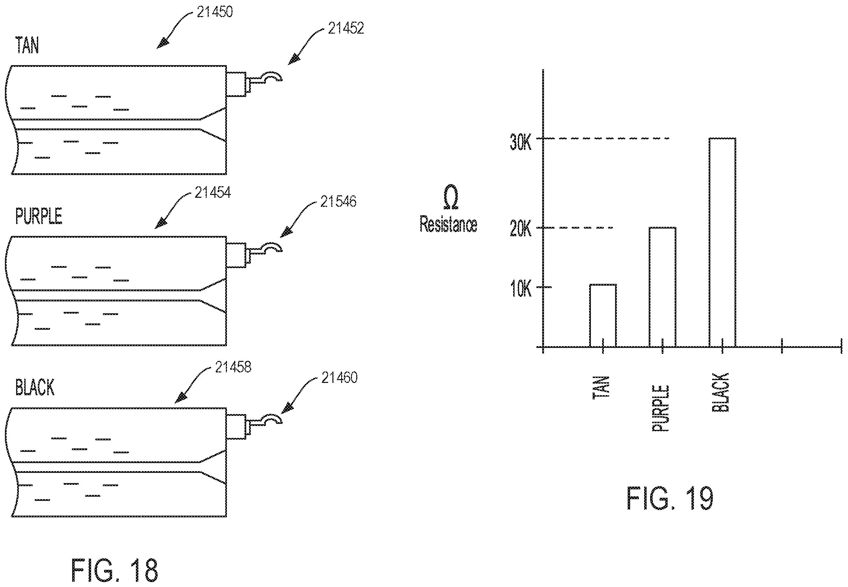

In addition to being able to determine if the staple cartridge is properly positioned in the cartridge channel, the receptacle assembly 21420 and the resistor assembly 21400 provides the added benefit of being able to determine the type of cartridge that is positioned in the cartridge channel, as referenced above. Once the control circuit is able to verify that the cartridge is properly positioned in the cartridge channel, by way of circuit 21428 and circuit 21406, an electrical signal can be transmitted to the circuit 21406 to determine a resistance of the resistor assembly 21400. As shown in FIGS. 18 and 19, a resistance determined from the resistor assembly can correspond to the color of the cartridge positioned within the cartridge channel, where the color of the cartridge can correspond to a variety of parameters of the staple cartridge, such as staple size, staple height, cartridge length, etc.

In one example, continuing to refer to FIGS. 18 and 19, when cartridge 21540 is positioned in cartridge channel, the control circuit can interrogate resistor assembly 21452 and sense that the resistance of the circuit therein is 10kΩ and determine that the cartridge is a tan staple cartridge that includes a plurality of staple cartridge parameters, such as cartridge length L1, staple height H1, etc. In another example, when cartridge 21544 is positioned in cartridge channel, the control circuit can interrogate resistor assembly 21456 and sense that the resistance of the circuit therein is 20kΩ and determine that the cartridge is a purple staple cartridge that includes a plurality of staple cartridge parameters, such as cartridge length L2, staple height H2, etc. In another example, when cartridge 21548 is positioned in cartridge channel, the control circuit can interrogate resistor assembly 21460 and sense that the resistance of the circuit therein is 30kΩ and determine that the corresponding staple cartridge is a black staple cartridge that includes a plurality of staple cartridge parameters, such as cartridge length L3, staple height H3, etc. While the above-provided discussion has been provided in the context of surgical stapling cartridges and staple cartridge parameters, it should be understood that the resistor assembly could be utilized in a plurality of other cartridge applications, such as RF cartridges, to determine the type of cartridge being attached to the surgical instrument.

The control circuit can be in electrical communicate with a display, such as other displays referenced herein, such that the control circuit can communicate information to a user of the surgical instrument. In one aspect, when the control circuit is able to verify that the cartridge is properly positioned in the cartridge channel, as described above with the circuits 21406, 21428, the control circuit can provide a visual indication that the cartridge properly coupled to the cartridge channel and is ready for use. Alternatively, the control circuit can cause audible or haptic feedback based on the cartridge properly coupled to the cartridge channel. After the control circuit identifies the type of cartridge positioned in the cartridge channel, the control circuit can display information about the cartridge onto the display, such as the color of the cartridge, the parameters of the cartridge, etc. In addition, after the control circuit identifies the type of cartridge positioned in the cartridge channel, the control circuit can modify parameters of the surgical instrument according to the parameters determined from the cartridge. For example, in instances where the control circuit identifies a cartridge with cartridge length L1, the cartridge can adjust a firing bar that traverses the cartridge to a suitable length for firing all of the staples from the cartridge, but not exceeding the length L1.

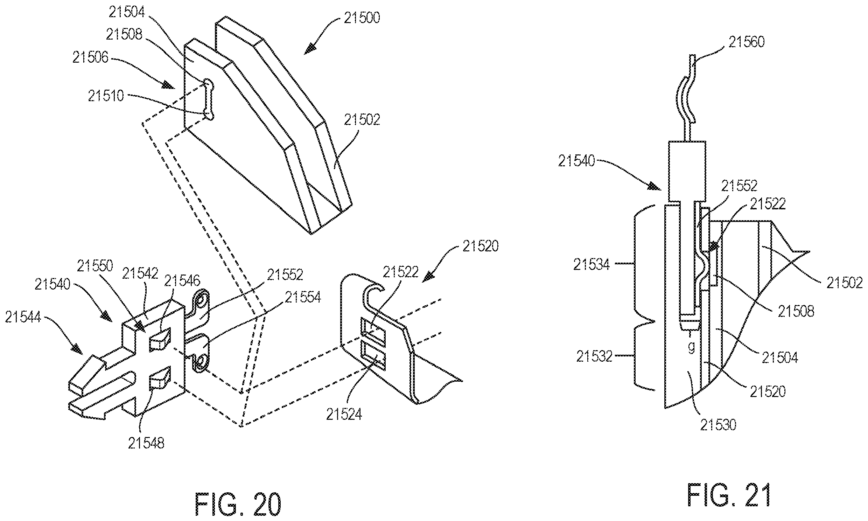

Referring now to FIGS. 20 and 21, a mechanism for determining if a staple cartridge is properly seated in a cartridge channel is provided, according to at least one aspect of the present disclosure. A staple cartridge can include a sled 21500 that can translate through the staple cartridge during a staple firing motion to deploy staples removably stored in the staple cartridge. In one aspect, the sled 21500 can include a plurality of ramps, such as an inner ramp 21502 and an outer ramp 51204 on a first lateral side of the staple cartridge, which are shaped to cam and deploy the staples from the staple cartridge during the firing stroke. The outer ramp 21504 of the sled 21500 can include an electrically printed circuit 21506 printed on an outer wall thereof. The circuit 21506 can include a first contact 21508 and a second contact 21510 in electrical communication with the first contact 21508.

The staple cartridge can further include a cartridge pan 21520 and an outer cartridge wall 21530. The cartridge pan 21520 can be sized to house the sled 21500 therein and can include a first window 21522 aligned with the first contact 21508 of the circuit 21506 and a second window 21524 aligned with the second contact 21510 of the circuit 21506. As shown in FIG. 21, the outer cartridge wall 21530 can at least partially abut the cartridge pan at an engagement region 21532 such that a gap 'g' can be defined between the cartridge wall 21530 and the cartridge pan 21520 in a connector receiving region 21534.

The connector receiving region 21534 and the gap 'g' can be sized to receive a connector assembly 21540 therein. The connector assembly 21540 can include a housing 21542, a connector portion 21544 extending from the housing 21542, a first window 21546, a second window 21548, and a circuit 21550 that can include a first contact arm 21552 that can extend proximally from the connector assembly 21540 and at least partially out of the first window 21546 and a second contact arm 21554 that can extend proximally from the connector assembly 21540 and at least partially out of the second window 21548. The proximal portions of the first contact arm 21552 and the second contact arm 21554 can be similar to the first contact arm 21409 and the second contact arm 21411, respectively, in that they are designed to electrically couple to a control circuit, such as control circuit 20014, as an example, located in the surgical instrument. For example, the surgical instrument can include circuit 21560, illustrated in FIG. 21, that can be in electrical communication with control circuit in the surgical instrument such that the control circuit can verify if the staple cartridge is properly positioned in the cartridge channel. Similarly, the connector assembly 21540 could include a circuit, similar to circuit 21406 in electrical communication with the first contact arm 21552 and the second contact arm 21554 such that the control circuit in the surgical instrument could determine a type of cartridge that the connector assembly 21540 is coupled to.

As shown in FIG. 21, when the connector assembly 21540 is properly positioned within the connector receiving region 21532 of the staple cartridge, the first circuit arm 21552 can extend through the first window 21546 of the connector assembly 21540, through the first window 21522 of the cartridge pan 21520, and can abut the first contact 21508 of the circuit 21506. Similarly, when the connector assembly 21540 is properly positioned within the connector receiving region 21532 of the staple cartridge, the second contact arm 21554 can extend through the second window 21548 of the connector assembly 21540, through the second window 21524 of the cartridge pan 21520, and can abut the second contact 21510 of the circuit 21506.

In operation, a user can determine if the connector assembly 21540 is properly coupled to the surgical instrument, by way of the proximal portions of the first contact arm 21552 and the second contact arm 21554 being electrically coupled with the circuit 21560, and if staple cartridge is properly seated within the cartridge channel, by way of the portions of the first contact arm 21552 and the second contact arm 21554 extending out of the first window 21546 and second window 21548, respectively, and electrically contacting the first contact 21508 and the second contact 21510. In one example, the control circuit can determine if the staple cartridge is properly coupled to the surgical instrument by generating an electrical signal that can transmit from the control circuit, through the circuit 21560, the first contact arm 21552, the circuit 21506, the second contact arm 21554 arm, the circuit 21560, and back to the control circuit. If the control circuit is unable to transmit an electrical signal from the control circuit as described above, a user will be able to determine that the connector assembly 21540 or the staple cartridge is improperly positioned and that corrective action is required.

The control circuit can be in electrical communication with a display, such as other displays referenced herein, such that the control circuit can communicate information to a user of the surgical instrument. In one aspect, when the control circuit is able to verify that the connector assembly 21540 and the staple cartridge are properly coupled to the surgical instrument, as described above, the control circuit can provide a visual indication verifying the same. Alternatively, the control circuit can cause audible or haptic feedback based on the control circuit verifying that the connector assembly 21540 and the staple cartridge are properly coupled to the surgical instrument.

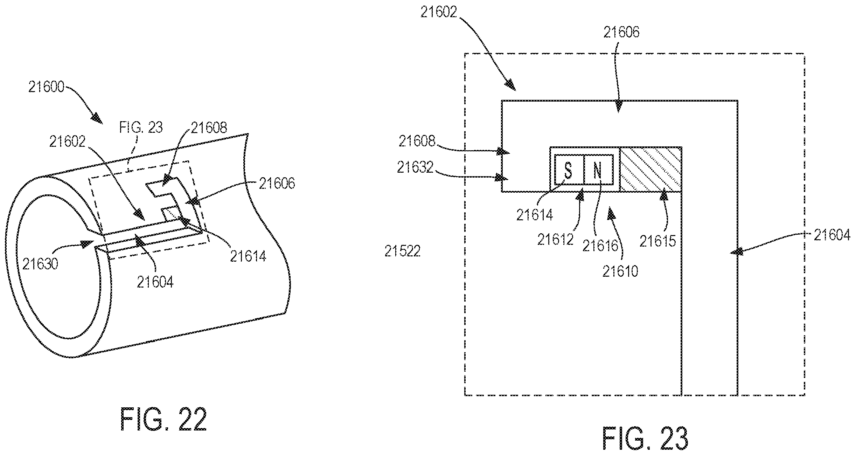

Referring now to FIGS. 22-29, a mechanism for ensuring that loading units are properly coupled to a surgical instrument is disclosed, according to at least one aspect of the present disclosure. As shown in FIG. 22, a shaft assembly 21600 can extend from a surgical housing assembly, such as a handle assembly or housing assembly. The shaft assembly 21600 can also be similar to other shaft assemblies described herein, such as shaft assembly 20005, shaft assembly 21104, shaft assembly 21200, and/or shaft assembly 21300, as non-limiting examples. The housing assembly can be similar to any other housing assemblies described herein, such as housing assembly 20001, housing assembly 21000, and/or housing assembly 21100, as non-limiting examples.

The shaft assembly 21600 can include a J-shaped passage 21602 defined therein. The J-shaped passage 21602 can include a first passage portion 21604, a second passage portion 21606 extending laterally away from the first passage portion 21602, and a third passage portion 21608 extending longitudinally away from the second passage portion 21606.

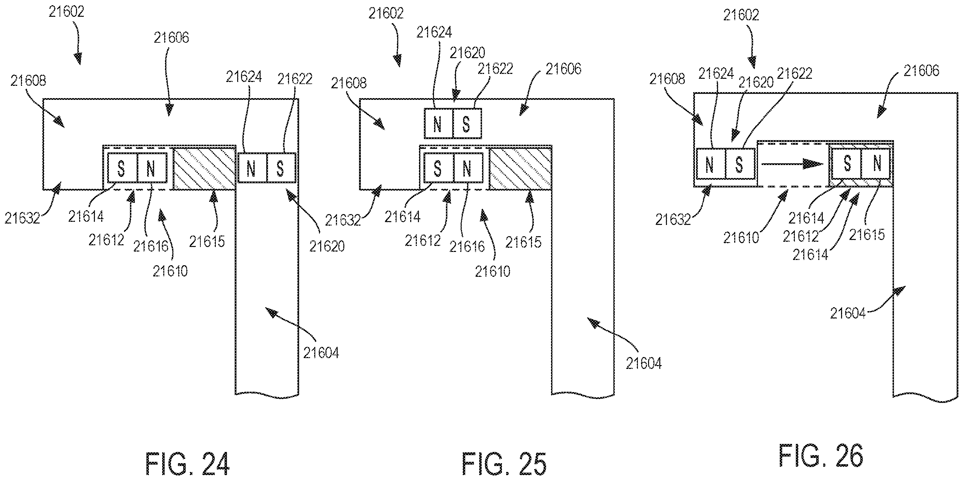

Referring primarily to FIG. 23, the shaft assembly 21600 can further include a closed-end tunnel 21610 positioned adjacent to the second passage portion 21606 and extending between the first passage portion 21604 and the second passage portion 21608. The closed-end tunnel 21610 can be sized to include a magnet 21612 therein that can be movable between a first position, as is shown in FIG. 23, where the magnet 21612 is positioned on a first end of the closed-end tunnel 21610 that is adjacent to the third passage portion 21608, and a second position, as is shown in FIG. 26, where the magnet 21612 is positioned on a second end of the closed-end tunnel 21610 that is adjacent to the first passage portion 21604. The shaft assembly 21600 can further include a window 21615 defined therein that allows a user to view the magnet 21612 when the magnet 21612 is in the second position.

As is shown in FIG. 23, the magnet 21612 can include a first magnet portion 21616 that includes a first polarity and a second magnet portion 21618 that includes a second polarity that is different than the first polarity. As an example, as shown in FIG. 23, the first magnet portion 21614 can include a south, negative polarity and the second magnet portion 21616 can include a north, positive polarity.

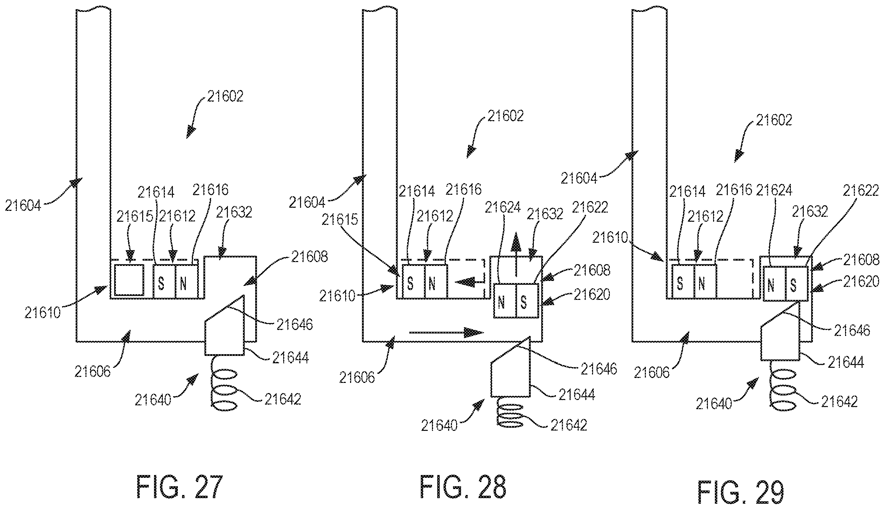

As referenced above, the above-provided mechanism can ensure that loading units, such as SULUs and/or MULUs, are properly coupled the shaft assembly 21600. Teferring to FIGS. 24-26, the loading unit can include a magnet 21620 coupled thereto. The magnet 21620 can include a first magnet portion 21622 that includes a first polarity, such as a south, negative polarity, and a second magnet portion 21624 that includes a second polarity that is different than the first polarity, such as a north, positive polarity. The first polarities of the magnets 216212, 21620 can be the same and the second polarities of the magnets 216212, 21620 can be the same. The loading unit can include a flange extending therefrom that includes the magnet coupled thereto. The flange can be sized to traverse through the J-shaped passage 21602 from the first passage portion 21604 to the third passage portion 21608. In order to lock the loading unit to the shaft assembly 21600, the magnet 21620 can move through the J-shaped passage 21602 and be positioned in the third passage portion 21608, as shown in FIGS. 26 and 29, as will be described in more detail below. In one aspect, the magnet 21620 being positioned in the third passage portion 21608 can correspond to the loading unit being locked to the shaft assembly 21600, therefore, allowing the user to know that the loading unit and the shaft assembly 21600 are safe for use with the surgical instrument.

In operation, as an example, the magnet 21620 of the loading unit can enter the first passage portion 21604 through an open-end 21630 of the J-shaped passage 21602 at a distal end of the shaft assembly 21600. The loading unit can be moved relative to the shaft assembly 21600 such that the magnet 21620 can be moved along the first passage portion 21604 toward the second passage portion 21606, as shown in FIG. 24. In one aspect, the magnet 21620 can be oriented such that the, as the magnet 21620 approaches the second passage portion 21620, the second polarities of the magnets 21612, 21620 can laterally align, as is shown in FIG. 24, causing the magnet 21612 to move to the first end of the closed-end tunnel 21640. In one aspect, when the magnet 21612 is on the first end of the closed-end tunnel 21610, the user is not able to view the magnet 21612 through the window 21615, therefore signifying that the loading unit is not yet coupled completely to the shaft assembly 21600.

Once the magnet 21620 has traversed the first passage portion 21604 and has reached the second passage portion 21606, the user can rotate the loading unit relative to the shaft assembly 21600 to traverse the magnet 21620 through the second passage portion 21606 toward the third passage portion 21608. As the magnet 21620 traverses the second passage portion 21606, the magnet 21620 can begin to longitudinally align with the magnet 21612 in the closed-end tunnel 21610, as is shown in FIG. 25. In one aspect, when the magnet 21620 begins to longitudinally align with magnet 21612, the first polarities of the magnets 21612, 21620 can begin to longitudinally align with the second polarities of the magnets 21612, 21620. The magnetic coupling force induced by the attraction between the polarities can cause the loading unit to experience resistance as the magnet 21620 is moved toward the third passage portion 21608. This magnetic arrangement can be utilized to reject immature attachments if the loading unit is incompletely attached to the shaft assembly 21600. With the magnets 21612, 21620 longitudinally aligned, a threshold force can be applied by the user to the loading unit to overcome the magnetic attractive forces between the magnets 21612, 21620 such that the magnet 21620 can continue to traverse the second passage portion 21606 toward the third passage portion 21608.

Once the magnet 21620 has reached the third passage portion 21608, the magnet 21602 can be moved to an end 21632 of the third passage portion 21608 that is adjacent to the first end of the closed-end passage 21610. As is shown in FIGS. 27-29, a spring assembly 21640 can be positioned at a transition point between the second passage portion 21606 and the third passage portion 21608. The spring assembly 21640 can include a spring 21642 coupled to the shaft assembly 21600 and a pusher plate 21644 coupled to the spring 21642. The spring 21642 can be transitionable between a compressed position, as shown in FIG. 28, where the pusher plate 21644 at least substantially is pushed out of the J-shaped passage 21602 and the spring 21642 is compressed, and an extended position, as shown in FIG. 29, where the pusher plate 21644 extends through the third passage portion 21608. The pusher plate 21644 can include a cam surface 21646 that can be engaged by the magnet 21620 as the magnet 21620 moves toward the third passage portion 21608 to transition the spring assembly 21640 toward the compressed position. As the magnet 21620 aligns with the third passage portion 21608, the user can release the loading unit, causing the spring assembly 21640 to transition toward the extended position, which can cause the pusher plate 21644 to force the magnet 21620 toward the end 21632 of the third passage portion 21608, as is shown in FIG. 29. The spring assembly 21640 can be designed such that, in the expanded position, the pusher plate 21644 can hold the magnet 21620 at the end 21632 of the third passage portion 21608 to maintain the loading unit locked and coupled to the shaft assembly 21600.

In one aspect, after the magnet 21620 overcomes the magnetic force experienced due to the magnet 21612, the second polarities of the magnets 21612, 21620 begin to approach one another, as is shown in FIG. 26, as an example, therefore causing the magnet 21612 to resist the magnet 21620. For example, as the magnet 21620 is moved toward the end 21632 of the third passage portion 21608, which can correspond to the loading unit being placed in the locked and coupled position with the shaft assembly 21600, magnetic resistance between the second polarities of the magnets 21612, 21620 can cause the magnet 21612 to move toward the second end of the closed-end channel 21610, as is shown in FIG. 26. As referenced above, when the magnet 21612 is in the second position at the second end of the closed-end channel 21610, a user is able to view the magnet 21612 through the window 21615, therefore signifying to the user that the magnet 21620 has reached the end 21632 of the third passage portion 21608 and that the loading unit is properly attached and coupled to the shaft assembly 21600.

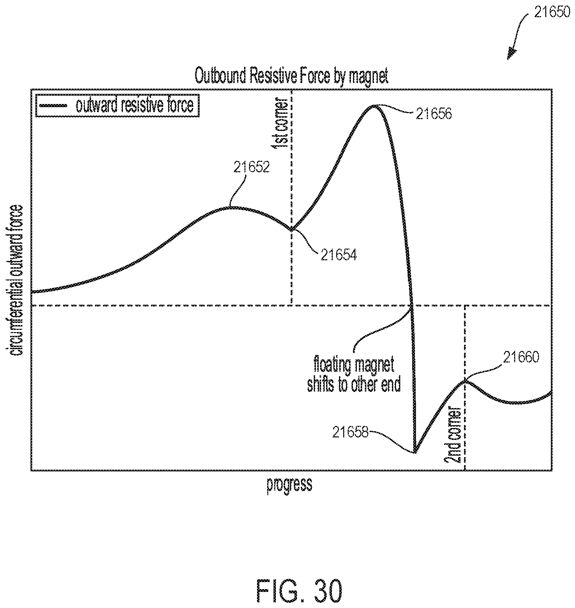

Referring now to FIG. 30, a graphical representation 21650 of the resistive force provided by the magnet 21612 as the magnet 21620 traverses the J-shaped passage 21602 is provided, according to at least one aspect of the present disclosure. A sensor assembly can be provided in the shaft assembly 21600 to measure magnetic forces between the magnets 21612, 21620 as the magnet 21620 traverses the J-shapes passage 21602. A control circuit located within the housing assembly, such as control circuit 20014, can be in electrical communication with the sensor assembly to monitor the magnetic forces between the magnets 21612, 21620 to provide feedback to a user indicative of the position of the magnet 21620 in the J-shapes passage 21602. The surgical instrument can include a display and the control circuit provide information to the user indicative of the magnetic force sensed by the sensor assembly via the display.

Initially, the magnet 21612 enters the open end 21630 of the J-shaped passage 21602 and traverses the first passage portion 21604 toward the second passage portion 21606. As the magnet 21620 traverses the first passage portion 21604 toward the second passage portion 21606, the circumferential outward force by the magnet 21620 can begin to increase until an inflection point 21652 is reached, where the magnet 21620 is laterally aligned with the magnet 21612, as is shown in FIG. 24, as an example.

After the magnet 21620 has laterally aligned with the magnet 21612, the magnet 21620 can continue to traverse the first passage portion 21604 toward the second passage portion 21606. As the magnetic moves toward the corner between first passage portion 21604 and the second passage portion 21606, the circumferential outward force by the magnet 21620 can diminish and reach inflection point 21654 when the magnet 21620 reaches the corner between the first passage portion 21606 and the second passage portion 21606.

After the magnet 21620 has reached the corner between the first passage portion 21606 and the second passage portion 21606, the magnet 21620 can traverse the second passage portion 21606 toward the third passage portion 21608. As the magnet 21620 traverses the second passage portion 21606 toward the third passage portion 21608, the circumferential outward force by the magnet 21612 begins to increase until an inflection point 21656 is reached, where the magnet 21620 is longitudinally aligned with the magnet 21612, as is shown in FIG. 25, as an example. As shown in FIG. 30, the inflection point 21565 force can be greater than the inflection point 21562.

After the magnet 21620 has longitudinally aligned with the magnet 21612, the magnet 21620 can continue to traverse the second passage portion 21606 toward the third passage portion 21608. As the magnet 21620 moves toward the corner between the second passage portion 21606 and the third passage portion 21608, the circumferential outward force by the magnet 21612 can shift as the phase change between the repulsion forces of the magnets 21612, 21620 changes from repulsive forces between the second polarities of the magnets 21612, 21620 (the north, positive polarities, as an example) to the first polarities of the magnets 21612, 21620 (the south, negative polarities, as an example). As the magnet 21620 moves toward the second corner between the second passage portion 21606 and the third passage portion 21608, the magnetic force between the magnets 21612, 21620, causes the magnet 21612 to translate toward the second end of the closed-end tunnel 21610, as is shown in FIG. 26, as an example.

As the magnet 21612 translates toward the second end of the closed-end tunnel 21610, the magnetic force can reach an inflection point 21658 and then can increase to inflection point 21660 as the magnet 21620 reaches the corner between the second passage portion 21606 and the third passage portion 21608. As the magnet 21620 then translates toward the end 21632 of the third passage portion 21608, the force can fluctuate as shown in FIG. 30 until the magnet 21620 reaches the end 21632 of the third passage portion 21608, where the loading unit is then locked to the shaft assembly 21600.

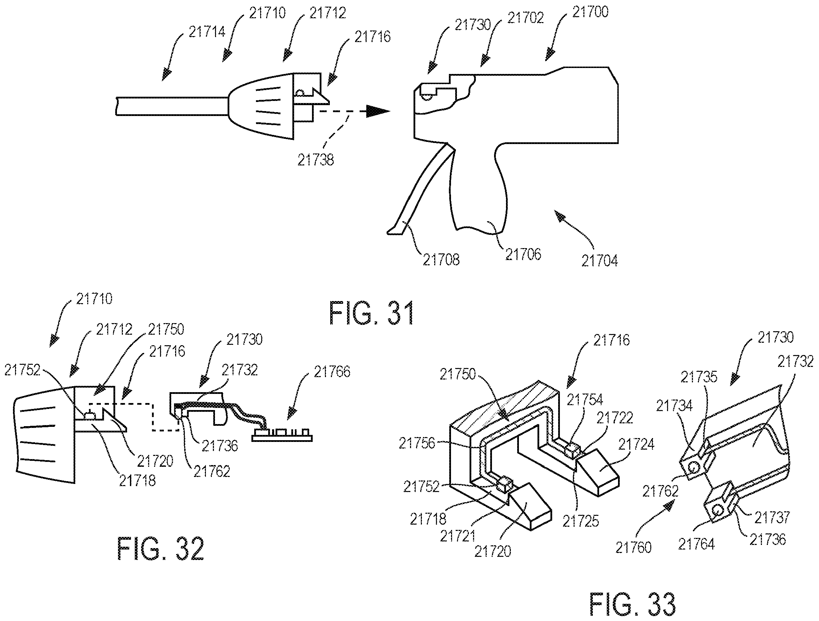

Referring now to FIGS. 31-33, a mechanism for determining if a nozzle assembly is properly coupled and completely installed with a handle assembly is provided, according to at least one aspect of the present disclosure. A handle assembly 21700 can include a housing portion 21702 and handle portion 21704. The handle portion 21704 can include a stationary handle 21706 and a trigger 21708 rotatable relative to the stationary handle 21706. The trigger 21708 can be rotatable toward the stationary handle 21706 to transmit actuation motions to an end effector of a loading unit, similar to as was described elsewhere herein. In one aspect, the trigger 21706 can transmit a closing motion that can cause a first jaw and a second jaw of the end effector to transition between an open configuration, wherein the first jaw and second jaw are spaced apart from one another, and a closed configuration, wherein the first jaw and second jaw are spaced near each other to capture tissue therebetween. In another aspect, the trigger 21708 can transmit a firing motion to the end effector to cause staples to be deployed from the end effector into the tissue positioned between the first jaw and second jaw, as well as cause a knife to sever the stapled tissue. The handle assembly can include more than one trigger than each effect different end effector functions of the end effector, such as closing motion and firing motions, as an example. The handle assembly 21700 can further include a control circuit, such as control circuit 21766, as an example, that can transmit electrical signals to various other components within the surgical instrument, such as to an end effector of a loading unit or a nozzle assembly 21710, as will be described in more detail below. The nozzle assembly 21710 can be similar to adapter assemblies described elsewhere herein, such as adapter 20002 and/or adapter 21002, as examples. The handle assembly 21700 can be similar to any other housing assemblies described herein, such as housing assembly 20001, housing assembly 21000 and/or housing assembly 21100, as non-limiting examples.

A nozzle assembly 21710 can include a nozzle housing 21712 that can be removably coupled to the handle housing 21702 and a shaft assembly 21714 extending distally from the nozzle housing 21712. The shaft assembly 21714 can be similar to other shaft assemblies described herein, such as shaft assembly 20005, shaft assembly 21104, shaft assembly 21200, shaft assembly 21300, and/or shaft assembly 21600, as non-limiting examples.

As shown in FIGS. 31-33, the nozzle assembly 21710 can include a nozzle latch 21716 extending proximally from the nozzle housing 21712. The nozzle latch 21716 can include a first seating platform or portion 21718 extending proximally from the nozzle housing 21712 and a first ramped portion 21720 extending proximally from the first seating portion 21718. Similarly, the nozzle latch 21716 can include a second seating platform or portion 21722 extending proximally from the nozzle housing 21712 and a second ramped portion 21724 extending proximally from the second seating portion 21722.

The handle assembly 21700 can include handle latch 21730 that includes a base portion 21732 and a pair of fingers 21734, 21736 extending transversely therefrom. In one aspect, to properly couple the nozzle assembly 21710 to the handle assembly 21700, the fingers 21734, 21736 can be positioned on correspondingly positioned seating portions 21718, 21722 to latch the nozzle assembly 21710 to the handle assembly 21700. Stated another way, to properly couple the nozzle assembly 21710 to the handle assembly 21700, finger 21734 can be seated on seating portion 21718 and finger 21736 can be seated on seating portion 21722.

In order to properly couple the nozzle assembly 21710 to the handle assembly 21700, the handle assembly 21700 can be brought towards the handle assembly 21700 in an installation direction 21738. As the nozzle assembly 21710 is brought towards the handle assembly 21700 in the installation direction 21738, finger 21734 can engage ramped portion 21720 and finger 21736 can engage ramped portion 21724 of the nozzle latch 21716. The fingers 21734, 21736 can slide along and cam the ramped portions 21720, 21724 downwardly away from the base portion 21732 of the handle latch 21730. As the fingers 21734, 21736 reach the apexes of the ramped portions 21720, 21724, the fingers 21724, 21736 can move distally and seat onto the seating portions 21718, 21722 of the nozzle latch 21716, respectively. As the fingers 21734, 21736 reach the seating portions 21718, 21722 of the nozzle latch 21716, the ramped portions 21720, 21724 can be biased such that the ramped portions 21720, 21724 return to their original, unbiased positions, as shown in FIG. 33, as an example. With the ramped portions 21720, 21724 in their original, unbiased positions and the fingers 21724, 21736 seated on the seating portions 21718, 21722, the distal surfaces 21721, 21725 of the ramped portions 21720, 21724 can engage the proximal surfaces 21735, 21737 of the fingers 21734, 21376, respectively, retaining the nozzle assembly 21710 to the handle assembly 21700, thereby properly coupling the nozzle assembly 21710 to the handle assembly 21700.

When the nozzle assembly 21710 is properly coupled to the handle assembly 21700, the handle assembly 21700 is capable of transmitting actuation motions and electrical signals through the nozzle assembly 21710 to an end effector at a distal end of the shaft assembly 21714, such as the aforementioned closure motions or firing motions, as an example. In situations where the nozzle assembly 21710 isn't properly coupled to the handle assembly 217100, the handle assembly 217100 may not be able to properly or safely transmit actuation motions or electrical signals to the end effector. In addition, in situations where the nozzle assembly 21700 isn't properly coupled to the handle assembly 21700, the nozzle assembly 21700 may decouple from the handle assembly 21700 during a surgical procedure, such as when the user attempts to transmit actuation motions to the end effector.

In order to ensure that the nozzle assembly 21710 is properly coupled to the handle assembly 21700, the nozzle latch 21716 can include a contact arrangement 21750 that includes a first latch contact 21752 positioned on the first seating portion 21718 and a second latch contact 21754 positioned on the second seating portion 21722. The first latch contact 21752 and the second latch contact 21754 can be in electrical communication by way of a wire 21756 that extends from the first latch contact 21752, along a distal, inner wall of the latch assembly 21716 and to the second latch contact 21756, as shown best in FIG. 33. In addition, the handle latch 21730 can include a contact arrangement 21760 that includes a first finger contact 21762 positioned on a bottom surface of the first finger 21734 and a second finger contact 21764 positioned on a bottom surface of the second finger 21736. The first finger contact 21762 and the second finger contact 21764 can be in electrical communication with the control circuit 21766 that is positioned in the handle assembly 21700.

In operation, when the nozzle assembly 21710 is coupled to the handle assembly 21700, as described above, the first finger contact 21762 can engage the first latch contact 21752 and the second finger contact 21764 can engage the second latch contact 21754. In order to verify if the nozzle assembly 21710 is properly coupled to the handle assembly 21700, the control circuit 21766 can attempt transmit an electrical signal through the contact arrangement 21760. In one aspect, if the control circuit 21766 is able to successfully transmit an electrical signal through the contact arrangement 21760, the control circuit 21766 can determine that the contact arrangement 21766 is in electrical communication with the contact arrangement 21750, signifying that the nozzle assembly 21710 is properly coupled to the handle assembly 21700. If the control circuit 21766 is unable to transmit an electrical signal through the contact arrangement 21760, the control circuit 21766 can determine that the nozzle assembly 21710 is improperly coupled to the handle assembly 21700 and that corrective action is required.

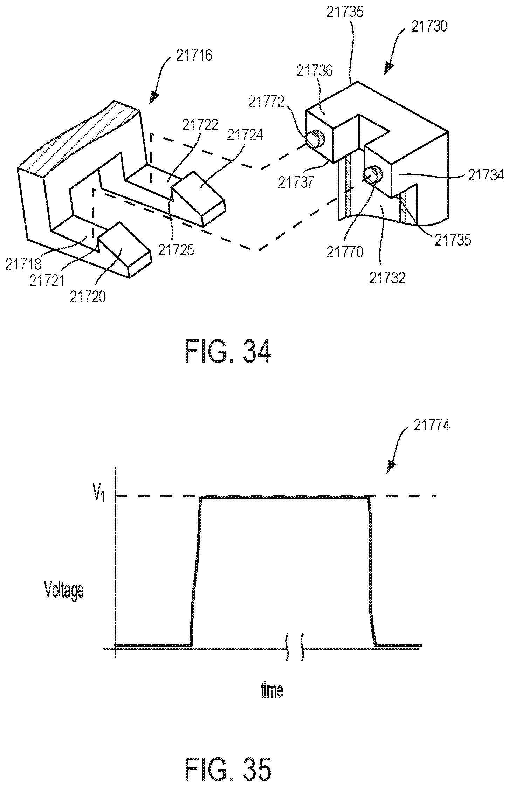

Alternatively, referring now to FIG. 34, the latch assembly 21716 may not include the contact arrangement 21750 and the latch assembly 21730 may include a first on-off switch 21770 and a second on-off switch 21772 on the first finger 21734 and the second finger 21736, respectively, in lieu of the first latch contact 21762 and the second contact 21764. The first on-off switch 21770 and the second on-off switch 21772 may be in electrical communication with a control circuit, such as control circuit 21766, which can determine an actuation state of the on-off switches 21770, 21772. The on-off switches 21770, 21772 can be transitionable between a resting position, as is shown in FIG. 34, which can signify to the control circuit that the fingers 21734, 21736 are not engaged with the seating portions 21718, 21722 of the latch assembly 21716, and an actuated position, which can signify to the control circuit that the fingers 21734, 21736 are engaged with the seating portions 21718, 21722 of the latch assembly 21716. The on-off switches 21770, 21772 can transition to the actuated position when the on-off switches 21770, 21772 are depressed toward the fingers 21734, 21736.

In operation, when the nozzle assembly 21710 is coupled to the handle assembly 21700, as described above, the first on-off switch 21770 can engage the first seating portion 21718 and the second on-off switch 21772 can engage the second seating portion 21722. In order to verify if the nozzle assembly 21710 is properly coupled to the handle assembly 21700, the contact circuit can monitor a voltage of the first on-off switch 21770 and the second on-off switch 21772. For example, referring to the graph 21774 in FIG. 35 that illustrates voltage sensed by the control circuit over time, when the nozzle assembly 21710 is not coupled to the handle assembly 21700, as is shown in FIG. 34, the on-off switches 21770, 21772 can be in the resting positions. The control circuit can sense that the on-off switches 21770, 21772 are in the resting position by measuring the voltage of the on-off switches to determine the position of the on-off switches 21770, 21772. As shown in FIG. 35, the control circuit senses a voltage of zero, therefore signifying to the control circuit that the nozzle assembly 21710 is not coupled to the handle assembly 21700. When the nozzle assembly 21710 is properly coupled to the handle assembly 21700, as described above, the control circuit can detect a voltage V₁ by the on-off switches 21770, 21772, thereby signifying that the nozzle assembly 21710 is properly coupled to the handle assembly 21700. If the nozzle assembly 21710 appears to be coupled to the handle assembly 21700, but the control circuit continues to detect a zero voltage, a user can determine that the nozzle assembly 21710 is not properly coupled to the handle assembly 21700 and that corrective action is required. The control circuit could detect a voltage that is greater than 0, but less than V₁. In such a scenario, the control circuit could determine that the first on-off switch 21770, as an example, is properly seated in the seating portion 21718, but on-off switch 21772 is not properly seated in the seating portion 21722, therefore resulting in a voltage detected by the control circuit that is less than V₁.

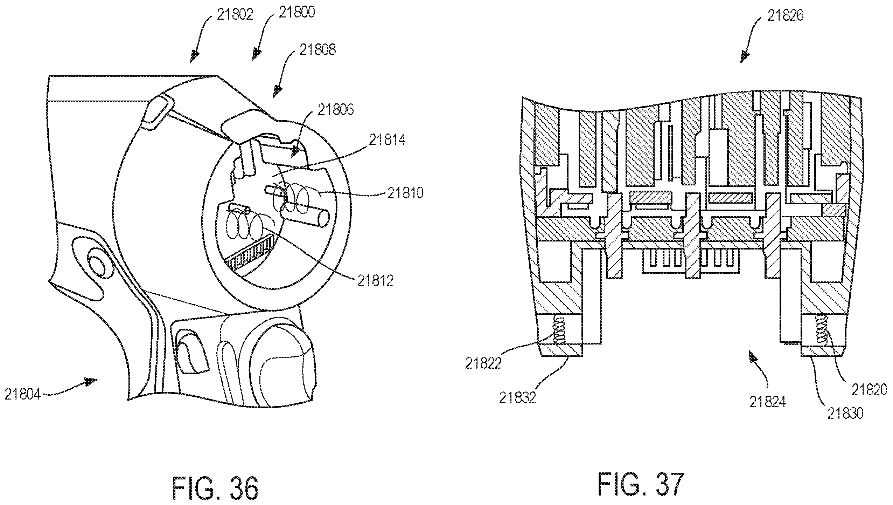

Referring now to FIG. 36, a mechanism for ensuring that an adapter is properly coupled and completely installed with a handle assembly is provided, according to at least one aspect of the present disclosure. A handle assembly 21800 can include a housing portion 21802 and handle portion 21804. The handle portion 21804 can be similar to other housing portions described herein, such as housing assembly 20001, housing assembly 21000, housing assembly 21100 and/or housing assembly 21700, as non-limiting examples.

In one aspect, the handle portion 21804 could include a stationary handle and one or more triggers that are rotatable relative to the stationary handle to effect end effector functions of a shaft assembly when the shaft assembly is properly coupled thereto. For example, when the shaft assembly is properly coupled to the handle assembly 21800, actuation of the triggers can cause the handle assembly 21800 to transmit actuation motions to the end effector of the shaft assembly, similar to what was described elsewhere herein. Actuation of one of the triggers could cause a closing motion that can cause a first jaw and a second jaw of the end effector to transition between an open configuration, wherein the first jaw and second jaw are spaced apart from one another, and a closed configuration, wherein the first jaw and second jaw are spaced near each other to capture tissue therebetween. Alternatively, actuation of one of the triggers could cause a firing motion to the end effector to cause staples to be deployed from the end effector into the tissue positioned between the first jaw and second jaw, as well as cause a knife to sever the stapled tissue.

The handle assembly 21800 can further include receiving area 21806 defined at a distal end 21808 thereof. The receiving area 21806 can be sized to receive a proximal end of an adapter assembly therein such that the handle assembly 21800 can transmit actuation motions and electrical signals through the adapter assembly. In one aspect, the receiving area can be similar to receiving area 21008 and adapter assembly can be similar to adapter assemblies described elsewhere herein, such as adapter 20002 and/or adapter 21002, as examples.

The receiving area 21806 can include a spring assembly that includes a first spring 21810 positioned on a first lateral side of a distal wall 21814 of the receiving area 21806 and a second spring 21812 positioned on a second lateral side of the distal wall 21816 of the receiving area 21806. The spring assembly could include only a single spring positioned at any suitable location of the receiving area 21806, such as in the center of the receiving area 21806. The spring assembly can include more than two springs positioned at any suitable locations of the receiving area 21806, such as around the perimeter of the distal wall 21814 of the receiving area 21806, as an example. The springs 21810, 21812 can be movable between an extended position, as shown in FIG. 36, and a compressed position, where the springs 21810, 21812 are compressed towards the distal wall 21814. In one aspect, the spring 21810, 21812 are linear springs and can be biased outwardly toward the extended position when no force is applied thereto. Alternatively, the springs 21810, 21812 can comprise torsional springs.

In one aspect, in order to properly and completely couple the adapter assembly to the handle assembly 21800, the proximal end of the adapter assembly can be moved into the receiving area 21806 to latch the adapter assembly to the housing assembly 21800. As one example, the adapter assembly can be latched to the handle assembly 21800 by way of flange features 21022a-e extending around the proximal end of the adapter assembly and flange features 21024a-e extending around the receiving area 21806, as described elsewhere herein. As the adapter assembly is moved into the receiving area 21806 to latch the shaft assembly to the handle housing 21802, the springs 21810, 21812 can abut the proximal end of the adapter assembly and apply a resistive force thereto. The springs 21810, 21812 can apply a resistive force to the adapter assembly such that the adapter assembly is biased away from the receiving area 21806 until the adapter assembly is latched to the handle assembly 21800.

The springs 21810, 21812 can provide a means of ensuring that the adapter assembly is properly coupled to the handle housing 21802 before the adapter assembly is utilizing in a surgical procedure. For example, should the flange features 21024a-e not completely or properly couple to the flange features 21022a-e, therefore signifying that the adapter assembly is properly coupled to the handle assembly 21800, the springs 21810, 21812 can force the adapter assembly away from the receiving area 21806. The springs 21810, 21812 therefore require that both a threshold force be applied to the adapter assembly to overcome the spring bias of the springs 21810, 21812, as well as also requires that the adapter assembly be properly coupled to the handle assembly 21800, otherwise the springs 21810, 21812 will force the adapter assembly away from the handle assembly 21800.

Referring now to FIG. 37, an alternative system is illustrated where springs 21820, 21822 can extend around a receiving area 21824 to bias a adapter assembly away from the receiving area 21826 unless the adapter assembly is properly coupled to a handle assembly 21826. In one aspect, spring 21822 can include a first platform 21830 coupled to spring 21822 and spring 21824 can include a second platform 21832 coupled to spring 21824. The platforms 21830, 21832 can increase the surface area to which the springs 21820, 21822 can apply the resistive force to the adapter assembly as the adapter assembly is brought into the receiving area 21824 of the handle assembly 21826.

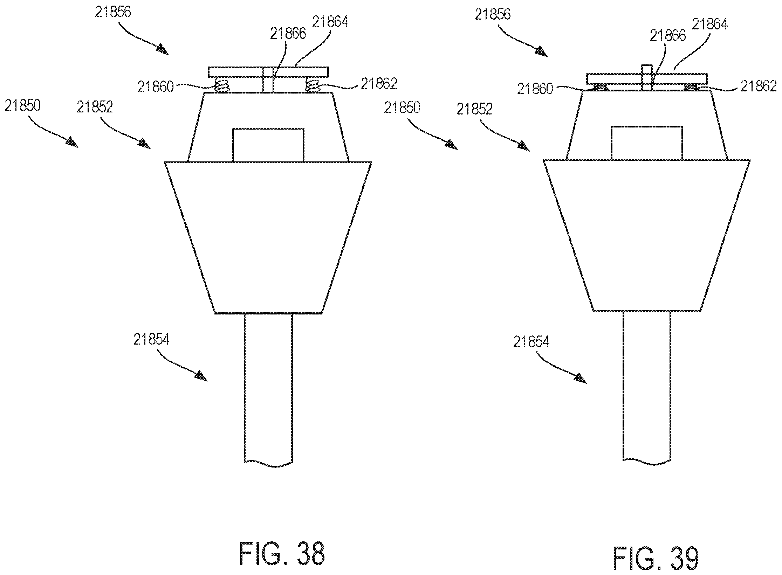

Referring now to FIGS. 38 and 39, another mechanism for ensuring that an adapter assembly is properly coupled and completely installed with a handle assembly is provided, according to at least one aspect of the present disclosure. An adapter assembly 21850 can include an adapter housing 21852 and a shaft 21854 extending distally therefrom. In one aspect, the adapter 21850 can be similar to adapter assembly 20002 and/or 21002, as examples. Similar to the above, the adapter assembly 21850 can be coupleable with a handle assembly by moving the proximal end 21856 of the adapter assembly 21850 into a receiving area of the handle assembly. Once the proximal end 21856 of the adapter assembly 21850 is properly positioned in the receiving area, a latch assembly, such as flange features 21022a-e and flange features 21024a-e, can lock the adapter assembly 21850 to the handle assembly.

Similar to the above, the adapter assembly 21850 can include a spring assembly that can include a first spring 21860 positioned on a first lateral side of the proximal end 21856 of the adapter assembly 21850 and a second spring 21862 positioned on a second lateral side of the proximal end 21856 of the adapter assembly 21850. The spring assembly can include more than two springs positioned at any suitable locations of the proximal end 21856 of the adapter assembly 21850, such as around the perimeter of the proximal end 21856 of the adapter assembly 21850, as an example. The springs 21860, 21862 can be movable between an extended position, as shown in FIG. 38, and a compressed position, as is shown in FIG. 39, where the spring 21860, 21862 are compressed towards the shaft 21854 of the adapter assembly 21850. In one aspect, the springs 21860, 21862 area linear springs and can be biased outwardly toward the extended position when no force is applied thereto.

As shown in FIGS. 38 and 39, the shaft assembly 21850 can further include a mounting plate 21864 that is coupled to the spring assembly. The mounting plate 21864 can be sized to be received within the receiving area of the housing assembly so as to align the adapter assembly 21850 with the handle assembly as the adapter assembly 21850 is coupled to the handle assembly. In addition, the adapter assembly 21850 can include an alignment shaft 21866 extending from the proximal end 21856 of the adapter assembly 21850 and through the mounting plate 21864. The alignment shaft 21864 can be sized to be received with an alignment aperture defined in the receiving area to assist in properly aligning the adapter assembly 21850 with the handle assembly as the adapter assembly 21850 is coupled to the handle assembly. The tip of the alignment shaft 21864 can be flush with the surface of the mounting plate 21864, as is shown in FIG. 38. As the mounting plate 21864 is pressed into the receiving area, the mounting plate 21864 can move toward the adapter assembly 21850 by way of the springs 21860, 21862 being compressed. As the mounting plate 21864 moves toward the shaft 21854, the alignment shaft 21864 can become exposed, as shown in FIG. 39, which can then move into the alignment aperture defined in the receiving area of the housing assembly to align the adapter assembly 21850 with the housing assembly.

As referenced above, as the adapter assembly 21850 is brought toward the handle assembly, the mounting plate 21864 and the alignment shaft 21866 can enter into the receiving area to assist in coupling the adapter assembly 21850 to the handle assembly. As the mounting plate 21864 is seated within the handle assembly, the springs 21860, 21862 can be compressed toward the compressed positions, as shown in FIG. 39. Similar to the above, the springs 21860, 21862 can apply a resistive force to bias the adapter assembly 21850 away from the mounting plate 21864. The springs 21860, 21862 can apply a resistive force to the adapter assembly 21850 such that the adapter assembly 21850 is biased away of the receiving area until the adapter assembly 21850 is latched to the handle assembly. The adapter assembly 21850 can be latched to the handle assembly when a portion of the adapter housing 21852 enters into the receiving area. For example, the adapter housing 21852 can include the plurality of flange features 21024a-e around the perimeter thereof such that, as the adapter housing 21852 enters the receiving area, the flange features 21024a-e can engage flange features 21024a-e of the receiving area of the housing assembly. Until flange features 21024a-e engage flange features 21024a-e to latch the adapter assembly 21850 to the housing assembly, the springs 21860, 21862 can bias the adapter assembly 21850 away from the receiving area.

The springs 21860, 21862 therefore provide a mechanism of ensuring that the adapter assembly 21850 is properly coupled to the handle housing before the adapter assembly 21850 is utilized in a surgical procedure. For example, should the flange features 21024a-e not completely or properly couple to the flange features 21022a-e, therefore signifying that the shaft assembly 21850 is not properly coupled to the handle assembly, the springs 21860, 21862 can force the shaft assembly 21850 away of the receiving area. The springs 21860, 21862 therefore require that both a threshold force be applied to the adapter assembly 21850 to overcome the spring bias of the springs 21860, 21862, as well as also requires that the adapter assembly 21850 be properly coupled to the handle assembly, otherwise the springs 21860, 21862 will force the adapter assembly 21850 away from the handle assembly.

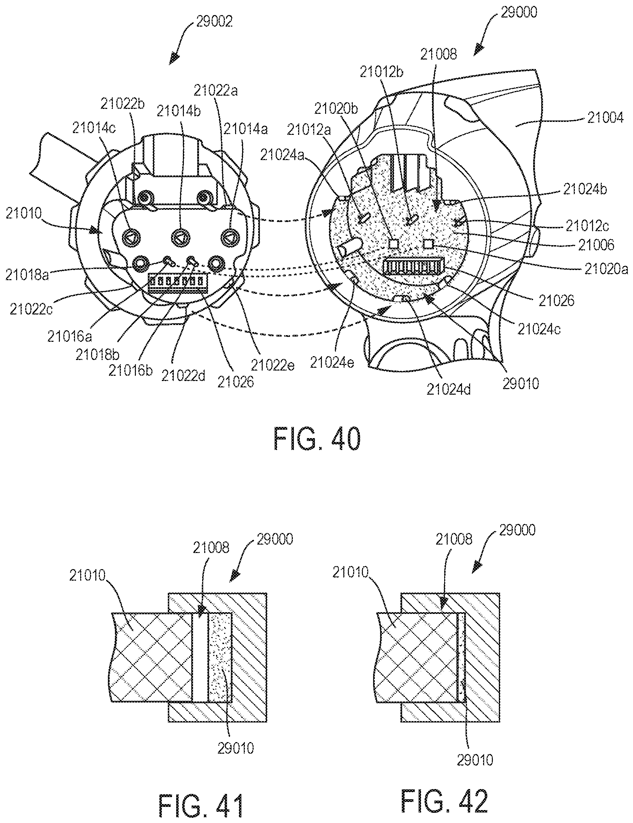

Referring now to FIG. 40, a housing 29000 and adapter 29002 are provided, in accordance with at least one aspect of the present disclosure. The housing 29000 and the adapter 29002 can substantially similar to housing assembly 21000 and adapter 21000 where like numbers are utilized to denote like features.

The recessed receiving area 21008 of the housing assembly 29000 can include a compliant material 29010 disposed therein. The compliant material 29010 can be positioned within the recessed receiving area 21008 such that the compliant material 29010 does not longitudinally overlap components of the housing assembly 29000 that interface with components of the adapter 29002, such as the contacts 21020a, 21020b, the electrical output connector 21026, the rotatable drive shafts 21012a, 21012b, 21012c, etc. Stated another way, the compliant material 29010 can occupy free space within the receiving area 21008 so as to take up any much surface area as possible without interfering in the adapters 29002 ability to properly couple to the housing 29000 and properly function.

The compliant material 29010 can comprise a compliant foam. The compliant material 29010 can comprise a compliant rubber. The compliant material 29010 can comprise a compliant lattice frame material. In one aspect, the compliant material 29010 is positioned within the receiving area 21008 such that, as the drive coupling assembly 21010 is moved into the receiving area 21008 to couple the adapter 29002 to the housing 29000, as described elsewhere herein, the compliant material 29010 can be deformed and resist the drive coupling assembly 21010 from moving proximally toward the latched orientation with the housing 29000.

For example, referring to FIG. 41, as the drive coupling assembly 21010 is moved toward the receiving area 21008 to latch the adapter 29002 to the housing 29000, the compliant material 29010 can be depressed by the drive coupling assembly 21010 and apply a resistive force to the drive coupling assembly 21010. The compliant material 29010 can be compressed by the drive coupling assembly 21010 as the flanges 21022a-e are moved towards the flanges 21024a-e, for example. In instances where the drive coupling assembly 21010 is not moved a sufficient amount relative to the housing such that the flanges 21022a-e engage flanges 21024a-e to couple the adapter 29002 to the housing 29000, the compliant material 29010 can expand and bias the drive coupling assembly 21010 away from the housing 29000. In one aspect, a user can need to apply a threshold force to the adapter 29002 so as to overcome the resistive force of the compliant material 29010 and compress the compliant material 29010 a sufficient amount, such as is shown in FIG. 42, in order to bring the flanges 21022a-e into operative engagement with the flanges 21204a-e to couple the adapter 29002 to the housing 29000. With the flanges 21022a-e in operative engagement with the flanges 21204a-e, the compliant material 29010 can be held compressed by the drive coupling assembly 21010, as shown in FIG. 41.

The above-provided compliant material 29010 can provide a means of ensuring that the adapter 29002 is properly coupled to the housing 29000 before the adapter 29002 is utilized in a surgical procedure. For example, should the flange features 21 024a-e not completely or properly couple to the flange features 21022a-e, therefore signifying that the adapter 29002 is not properly coupled to the housing 29000, the compliant material 29010 can force the adapter 29002 away from the housing 29000. The compliant material 29010 therefore requires that a threshold force be applied to the adapter 29002 to overcome the compliant material 29010 resistive bias, otherwise the compliant material will force the adapter 29002 away from housing 29000.

It should be understood any of the foregoing systems can be utilized in connection with one another so that a user would be capable of detecting irregularities and incomplete connections at various positions throughout the surgical instrument. For example, a surgical instrument according to the invention includes a detector assembly for determining if a shaft assembly is properly connected to a loading unit, and could include a detector assembly for determining if an adapter is properly coupled to a handle assembly, and a detector assembly for determining if an end effector and/or cartridge is properly coupled to the surgical instrument. Each of the detection assemblies can include their own dedicated electrical arrangement and be coupled to the control circuit positioned within the handle assembly such that the control circuit can identify the position of the incomplete connection within the surgical instrument. In an instance where the control circuit identifies an incomplete connection within the surgical instrument using any of the foregoing mechanisms disclosed herein, the control circuit can provide feedback to user indicative of the location of the incomplete connection. For example, the control circuit can cause a display to display a location of the incomplete connection detected by any of the foregoing mechanisms disclosed herein.

Numerous specific details are set forth to provide a thorough understanding of the overall structure, function, manufacture, and use of the systems as described in the specification and illustrated in the accompanying drawings. Well-known operations, components, and elements have not been described in detail so as not to obscure the systems described in the specification. The reader will understand that the systems described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other systems are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other systems are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other systems are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

Although various devices have been described herein in connection with certain systems, modifications and variations to those systems may be implemented. Particular features, structures, or characteristics may be combined in any suitable manner in one or more systems. Thus, the particular features, structures, or characteristics illustrated or described in connection with one system may be combined in whole or in part, with the features, structures or characteristics of one or more other systems without limitation. Also, where materials are disclosed for certain components, other materials may be used. Furthermore, according to various systems, a single component may be replaced by multiple components, and multiple components may be replaced by a single component, to perform a given function or functions.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the disclosure, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

As discussed above, the surgical instruments disclosed herein may comprise control systems. Each of the control systems can comprise a circuit board having one or more processors and/or memory devices. Among other things, the control systems are configured to store sensor data, for example. They are also configured to store data which identifies the type of staple cartridge attached to a stapling instrument, for example. More specifically, the type of staple cartridge can be identified when attached to the stapling instrument by the sensors and the sensor data can be stored in the control system. This information can be obtained by the control system to assess whether or not the staple cartridge is suitable for use.

The surgical instrument systems described herein are motivated by an electric motor; however, the surgical instrument systems described herein can be motivated in any suitable manner. In certain instances, the motors disclosed herein may comprise a portion or portions of a robotically controlled system. U.S. Patent Application Serial No. 13/118,241, entitled SURGICAL STAPLING INSTRUMENTS WITH ROTATABLE STAPLE DEPLOYMENT ARRANGEMENTS, now U.S. Patent No. 9,072,535, for example, discloses several examples of a robotic surgical instrument system in greater detail.

The term "substantially", "about", or "approximately" as used in the present disclosure, unless otherwise specified, means an acceptable error for a particular value as determined by one of ordinary skill in the art, which depends in part on how the value is measured or determined. The term "substantially", "about", or "approximately" can mean within 1, 2, 3, or 4 standard deviations. The term "substantially", "about", or "approximately" can mean within 50%, 20%, 15%, 10%, 9%, 8%, 7%, 6%, 5%, 4%, 3%, 2%, 1%, 0.5%, or 0.05% of a given value or range.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. A surgical system, comprising:
a housing assembly (20001) comprising an elongate shaft (21200) extending therefrom, wherein the housing assembly (20001) comprises a first contact;
a loading unit (21202) removably coupleable to the elongate shaft (21200), wherein the loading unit (21202) comprises a second contact, wherein the first contact is configured to electrically communicate with the second contact based on the loading unit (21202) being coupled to the elongate shaft (21200); and
a first detector assembly configured to determine if the loading unit (21202) is coupled to the elongate shaft (21200); **characterized in that**:
the loading unit (21202) is rotatable relative to the elongate shaft (21200) between an unlocked position, wherein the loading unit (21202) is movable away from the housing assembly (20001), and a locked position, wherein the loading unit (21202) is coupled to the elongate shaft (21200);
wherein the first detector assembly comprises either:
(a) a first magnet (21220) coupled to the loading unit (21202), wherein the first magnet (21220) comprises a first polarity;
a second magnet (21222) coupled to the loading unit (21202), wherein the second magnet (21222) comprises a second polarity different than the first polarity; and
a sensor (21226) coupled to the elongate shaft (21200), wherein the sensor (21226) is configured to detect the first polarity of the first magnet (21220) when the loading unit (21202) is in the unlocked position, and wherein the sensor (21226) is configured to detect the second polarity of the second magnet (21222) when the loading unit (21202) is in the locked position; or
(b) a first capacitor (21132) coupled to the loading unit (21202);
a second capacitor (21130) coupled to the elongate shaft (21200); and
a sensor configured to detect a capacitance between the first capacitor (21132) and the second capacitor (21130), wherein the sensor is configured to sense a first capacitance between the first capacitor (21132) and the second capacitor (21130) based on the loading unit (21202) being in the unlocked position, and wherein the sensor is configured to sense a second capacitance between the first capacitor (21132) and the second capacitor (21130) based on the loading unit (21202) being in the locked position.

2. The surgical system of Claim 1, option (a), wherein the sensor (21226) comprises a Hall-effect sensor.

3. The surgical system of Claim 1 or 2, wherein the first detector assembly comprises:
a lug (21310, 21312) extending from the loading unit (21302); and
a spring assembly (21314) coupled to the elongate shaft (21300).

4. The surgical system of Claim 3, wherein the spring assembly (21314) is configured to resist rotation of the loading unit (21302) as the loading unit (21302) is rotated toward the locked position.

5. The surgical system of Claim 4, wherein the spring assembly (21314) is prevented from rotating the loading unit (21302) toward the unlocked position upon the loading unit (21302) reaching the locked position.

6. The surgical system of claim 1, wherein:
the housing assembly comprise a handle assembly (29000) and an adapter assembly (29002) removably coupleable to the handle assembly (29000), wherein the adapter assembly (29002) comprises the elongate shaft (21300) extending therefrom; and further comprising:
a second detector assembly configured to determine if the adapter assembly (29002) is coupled to the handle assembly (29000).

7. The surgical system of Claim 6, wherein the second detector assembly comprises compliant material (29010) positioned at a coupling interface between the handle assembly (29000) and the adapter assembly (29002), wherein the compliant material (29010) is configured to bias the adapter assembly (29002) away from the handle assembly (29000) absent the adapter assembly (29002) being coupled to the handle assembly (29000).

8. The surgical system of Claim 6 or 7, wherein the second detector assembly comprises:
a first latch (21716) coupled to the adapter assembly (29002), wherein the first latch (21716) comprises:
a first seating platform (21718) comprising a first seating contact (21752); and
a second seating platform (21722) comprising a second seating contact (21754), wherein the first seating contact (21752) is in electrical communication with the second seating contact (21754); and
a second latch (21730) coupled to the handle assembly (29000), wherein the second latch (21730) is configured to engage the first latch (21716) to couple the adapter assembly (29002) to the handle assembly (29000), and wherein the second latch (21730) comprises:
a first finger (21734) comprising a first finger contact (21762), wherein the first finger contact (21762) is configured to electrically couple to the first seating contact (21752) based on the first finger (21734) engaging the first seating platform (21718); and
a second finger (21736) comprising a second finger contact (21764), wherein the second finger contact (21764) is configured to electrically couple to the second seating contact (21754) based on the second finger (21736) engaging the second seating platform (21722).

9. The surgical system of Claim 6 or 7 wherein the second detector assembly comprises:
a first latch (21716) coupled to the adapter assembly (29002), wherein the first latch (21716) comprises:
a first seating platform (21718); and
a second seating platform (21722); and
a second latch (21730) coupled to the handle assembly (29000), wherein the second latch (21730) is configured to engage the first latch (21716) to couple the adapter assembly (29002) to the handle assembly (29000), and wherein the second latch (21730) comprises:
a first finger (21734) comprising a first on-off switch (21770), wherein the first on-off switch (21770) is configured to actuate based on the first on-off switch (21770) engaging the first seating platform (21718); and
a second finger (21736) comprising a second on-off switch (21772), wherein the second on-off switch (21772) is configured to actuate based on the second on-off switch (21772) engaging the second seating platform (21722).

10. The surgical system of Claim 6, wherein the second detector assembly comprises:
a third contact (21020a) disposed on the handle assembly (29000);
a fourth contact (21020b) disposed on the handle assembly (29000);
a first depressible shaft (21016a) extending from the adapter assembly (29002), wherein the first depressible shaft (21016a) is configured to engage the third contact (21020a); and
a second depressible shaft (21016b) extending from the adapter assembly (29002), wherein the second depressible shaft (21016b) is configured to engage the fourth contact (21020b).

## Patentansprüche

1. Chirurgisches System, umfassend:
eine Gehäuseanordnung (20001), die einen davon ausgehenden länglichen Schaft (21200) umfasst, wobei die Gehäuseanordnung (20001) einen ersten Kontakt umfasst;
eine Ladeeinheit (21202), die abnehmbar mit dem länglichen Schaft (21200) gekoppelt werden kann, wobei die Ladeeinheit (21202) einen zweiten Kontakt umfasst, wobei der erste Kontakt konfiguriert ist, um basierend darauf, dass Ladeeinheit (21202) mit dem länglichen Schaft (21200) gekoppelt ist, elektrisch mit dem zweiten Kontakt zu kommunizieren; und
eine erste Detektoranordnung, die konfiguriert ist, um zu bestimmen, ob die Ladeeinheit (21202) mit dem länglichen Schaft (21200) gekoppelt ist; **dadurch gekennzeichnet, dass**:
die Ladeeinheit (21202) relativ zu dem länglichen Schaft (21200) zwischen einer entriegelten Position, in der die Ladeeinheit (21202) von der Gehäuseanordnung (20001) weg bewegt werden kann, und einer verriegelten Position, in der die Ladeeinheit (21202) mit dem länglichen Schaft (21200) gekoppelt ist, drehbar ist;
wobei die erste Detektoranordnung entweder umfasst:
(a) einen ersten Magneten (21220), der mit der Ladeeinheit (21202) gekoppelt ist, wobei der erste Magnet (21220) eine erste Polarität aufweist;
einen zweiten Magneten (21222), der mit der Ladeeinheit (21202) gekoppelt ist, wobei der zweite Magnet (21222) eine zweite Polarität aufweist, die sich von der ersten Polarität unterscheidet; und
einen Sensor (21226), der mit dem länglichen Schaft (21200) gekoppelt ist, wobei der Sensor (21226) konfiguriert ist, um die erste Polarität des ersten Magneten (21220) zu erkennen, wenn sich die Ladeeinheit (21202) in der entriegelten Position befindet, und wobei der Sensor (21226) konfiguriert ist, um die zweite Polarität des zweiten Magneten (21222) zu erkennen, wenn sich die Ladeeinheit (21202) in der verriegelten Position befindet; oder
(b) einen ersten Kondensator (21132), der mit der Ladeeinheit (21202) gekoppelt ist;
einen zweiten Kondensator (21130), der mit dem länglichen Schaft (21200) gekoppelt ist; und
einen Sensor, der konfiguriert ist, um eine Kapazität zwischen dem ersten Kondensator (21132) und dem zweiten Kondensator (21130) zu erkennen, wobei der Sensor konfiguriert ist, um eine erste Kapazität zwischen dem ersten Kondensator (21132) und dem zweiten Kondensator (21130) basierend darauf zu erfassen, dass sich die Ladeeinheit (21202) in der entriegelten Position befindet, und wobei der Sensor konfiguriert ist, um eine zweite Kapazität zwischen dem ersten Kondensator (21132) und dem zweiten Kondensator (21130) basierend darauf zu erfassen, dass sich die Ladeeinheit (21202) in der verriegelten Position befindet.

2. Chirurgisches System nach Anspruch 1, Option (a), wobei der Sensor (21226) einen Hall-Effekt-Sensor umfasst.

3. System nach Anspruch 1 oder 2, wobei die erste Detektoranordnung umfasst:
eine Lasche (21310, 21312), die von der Ladeeinheit (21302) ausgeht; und
eine Federanordnung (21314), die mit dem länglichen Schaft (21300) gekoppelt ist.

4. Chirurgisches System nach Anspruch 3, wobei die Federanordnung (21314) konfiguriert ist, einer Drehung der Ladeeinheit (21302) Widerstand entgegenzusetzen, wenn die Ladeeinheit (21302) in Richtung der verriegelten Position gedreht wird.

5. Chirurgisches System nach Anspruch 4, wobei die Federanordnung (21314) daran gehindert wird, die Ladeeinheit (21302) in Richtung der entriegelten Position zu drehen, wenn die Ladeeinheit (21302) die verriegelte Position erreicht.

6. Chirurgisches System nach Anspruch 1, wobei:
die Gehäuseanordnung eine Griffanordnung (29000) und eine Adapteranordnung (29002), die abnehmbar mit der Griffanordnung (29000) gekoppelt werden kann, umfasst, wobei die Adapteranordnung (29002) den davon ausgehenden länglichen Schaft (21300) umfasst; und ferner umfassend:
eine zweite Detektoranordnung, die konfiguriert ist, um zu bestimmen, ob die Adapteranordnung (29002) mit der Griffanordnung (29000) gekoppelt ist.

7. Chirurgisches System nach Anspruch 6, wobei die zweite Detektoranordnung nachgiebiges Material (29010) umfasst, das an einer Kopplungsschnittstelle zwischen der Griffanordnung (29000) und der Adapteranordnung (29002) positioniert ist, wobei das nachgiebige Material (29010) konfiguriert ist, um die Adapteranordnung (29002) von der Griffanordnung (29000) weg vorzuspannen, wenn die Adapteranordnung (29002) nicht mit der Griffanordnung (29000) gekoppelt ist.

8. Chirurgisches System nach Anspruch 6 oder 7, wobei die zweite Detektoranordnung umfasst:
einen ersten Riegel (21716), der mit der Adapteranordnung (29002) gekoppelt ist, wobei der erste Riegel (21716) umfasst:
eine erste Sitzplattform (21718), die einen ersten Sitzkontakt (21752) umfasst; und
eine zweite Sitzplattform (21722), die einen zweiten Sitzkontakt (21754) umfasst, wobei der erste Sitzkontakt (21752) in elektrischer Verbindung mit dem zweiten Sitzkontakt (21754) steht; und
einen zweiten Riegel (21730), der mit der Griffanordnung (29000) gekoppelt ist, wobei der zweite Riegel (21730) konfiguriert ist, um mit dem ersten Riegel (21716) in Eingriff zu treten, um die Adapteranordnung (29002) mit der Griffanordnung (29000) zu koppeln, und wobei der zweite Riegel (21730) umfasst:
einen ersten Finger (21734), der einen ersten Fingerkontakt (21762) umfasst, wobei der erste Fingerkontakt (21762) konfiguriert ist, um sich basierend darauf, dass der erste Finger (21734) mit der ersten Sitzplattform (21718) in Eingriff steht, elektrisch mit dem ersten Sitzkontakt (21752) zu koppeln; und
einen zweiten Finger (21736), der einen zweiten Fingerkontakt (21764) umfasst, wobei der zweite Fingerkontakt (21764) konfiguriert ist, um sich basierend darauf, dass der zweite Finger (21736) mit der zweiten Sitzplattform (21722) in Eingriff steht, elektrisch mit dem zweiten Sitzkontakt (21754) zu koppeln.

9. Chirurgisches System nach Anspruch 6 oder 7, wobei die zweite Detektoranordnung umfasst:
einen ersten Riegel (21716), der mit der Adapteranordnung (29002) gekoppelt ist, wobei der erste Riegel (21716) umfasst:
eine erste Sitzplattform (21718); und
eine zweite Sitzplattform (21722); und
einen zweiten Riegel (21730), der mit der Griffanordnung (29000) gekoppelt ist, wobei der zweite Riegel (21730) konfiguriert ist, um mit dem ersten Riegel (21716) in Eingriff zu treten, um die Adapteranordnung (29002) mit der Griffanordnung (29000) zu koppeln, und wobei der zweite Riegel (21730) umfasst:
einen ersten Finger (21734), der einen ersten Ein-/Ausschalter (21770) umfasst, wobei der erste Ein-/Ausschalter (21770) konfiguriert ist, um basierend darauf, dass der erste Ein-/Ausschalter (21770) mit der ersten Sitzplattform (21718) in Eingriff tritt, auszulösen; und
einen zweiten Finger (21736), der einen zweiten Ein-/Ausschalter (21772) umfasst, wobei der zweite Ein-/Ausschalter (21772) konfiguriert ist, um basierend darauf, dass der zweite Ein-/Ausschalter (21772) mit der zweiten Sitzplattform (21722) in Eingriff tritt, auszulösen.

10. Chirurgisches System nach Anspruch 6, wobei die zweite Detektoranordnung umfasst:
einen dritten Kontakt (21020a), der an der Griffanordnung (29000) angeordnet ist;
einen vierten Kontakt (21020b), der an der Griffanordnung (29000) angeordnet ist;
einen ersten eindrückbaren Schaft (21016a), der von der Adapteranordnung (29002) ausgeht, wobei der erste eindrückbare Schaft (21016a) konfiguriert ist, um mit dem dritten Kontakt (21020a) in Eingriff zu treten; und
einen zweiten eindrückbaren Schaft (21016b), der von der Adapteranordnung (29002) ausgeht, wobei der zweite eindrückbare Schaft (21016b) konfiguriert ist, um mit dem vierten Kontakt (21020b) in Eingriff zu treten.

## Revendications

1. Système chirurgical, comprenant :
un ensemble boîtier (20001) comprenant un arbre allongé (21200) s'étendant à partir de celui-ci, dans lequel l'ensemble boîtier (20001) comprend un premier contact ;
une unité de chargement (21202) pouvant être couplée de manière amovible à l'arbre allongé (21200), dans lequel l'unité de chargement (21202) comprend un deuxième contact, dans lequel le premier contact est configuré pour communiquer électriquement avec le deuxième contact lorsque l'unité de chargement (21202) est couplée à l'arbre allongé (21200) ; et
un premier ensemble détecteur configuré pour déterminer si l'unité de chargement (21202) est couplée à l'arbre allongé (21200) ;
**caractérisé en ce que** :
l'unité de chargement (21202) peut tourner par rapport à l'arbre allongé (21200) entre une position déverrouillée, dans lequel l'unité de chargement (21202) peut s'éloigner de l'ensemble boîtier (20001), et une position verrouillée, dans lequel l'unité de chargement (21202) est couplée à l'arbre allongé (21200) ;
dans lequel le premier ensemble détecteur comprend soit :
(a) un premier aimant (21220) couplé à l'unité de chargement (21202), dans lequel le premier aimant (21220) comprend une première polarité ;
un second aimant (21222) couplé à l'unité de chargement (21202), dans lequel le second aimant (21222) comprend une seconde polarité différente de la première polarité ; et
un capteur (21226) couplé à l'arbre allongé (21200), dans lequel le capteur (21226) est configuré pour détecter la première polarité du premier aimant (21220) lorsque l'unité de chargement (21202) est dans la position déverrouillée, et dans lequel le capteur (21226) est configuré pour détecter la seconde polarité du second aimant (21222) lorsque l'unité de chargement (21202) est dans la position verrouillée ; soit
(b) un premier condensateur (21132) couplé à l'unité de chargement (21202) ;
un second condensateur (21130) couplé à l'arbre allongé (21200) ; et
un capteur configuré pour détecter une capacité entre le premier condensateur (21132)
et le second condensateur (21130), dans lequel le capteur est configuré pour détecter une première capacité entre le premier condensateur (21132) et le second condensateur (21130) lorsque l'unité de chargement (21202) est dans la position déverrouillée, et dans lequel le capteur est configuré pour détecter une seconde capacité entre le premier condensateur (21132) et le second condensateur (21130) lorsque l'unité de chargement (21202) est dans la position verrouillée.

2. Système chirurgical selon la revendication 1, option (a), dans lequel le capteur (21226) comprend un capteur à effet Hall.

3. Système chirurgical selon la revendication 1 ou 2, dans lequel le premier ensemble détecteur comprend :
un ergot (21310, 21312) s'étendant à partir de l'unité de chargement (21302) ; et
un ensemble ressort (21314) accouplé à l'arbre allongé (21300).

4. Système chirurgical selon la revendication 3, dans lequel l'ensemble ressort (21314) est conçu pour résister à la rotation de l'unité de chargement (21302) lorsque l'unité de chargement (21302) est tournée vers la position verrouillée.

5. Système chirurgical selon la revendication 4, dans lequel l'ensemble ressort (21314) est empêché de faire tourner l'unité de chargement (21302) vers la position déverrouillée lorsque l'unité de chargement (21302) atteint la position verrouillée.

6. Système chirurgical selon la revendication 1, dans lequel :
l'ensemble boîtier comprend un ensemble poignée (29000) et un ensemble adaptateur (29002) pouvant être couplé de manière amovible à l'ensemble poignée (29000), dans lequel l'ensemble adaptateur (29002) comprend l'arbre allongé (21300) s'étendant à partir de celui-ci ; et comprenant en outre :
un second ensemble détecteur configuré pour déterminer si l'ensemble adaptateur (29002) est couplé à l'ensemble poignée (29000).

7. Système chirurgical selon la revendication 6, dans lequel le second ensemble détecteur comprend un matériau conforme (29010) positionné à une interface de couplage entre l'ensemble poignée (29000) et l'ensemble adaptateur (29002), dans lequel le matériau conforme (29010) est conçu pour éloigner l'ensemble adaptateur (29002) de l'ensemble de poignée (29000) en l'absence de l'ensemble adaptateur (29002) couplé à l'ensemble de poignée (29000).

8. Système chirurgical selon la revendication 6 ou 7, dans lequel le second ensemble détecteur comprend :
un premier loquet (21716) accouplé à l'ensemble adaptateur (29002), dans lequel le premier loquet (21716) comprend :
une première plate-forme d'assise (21718) comprenant un premier contact d'assise (21752) ; et
une seconde plate-forme d'assise (21722) comprenant un second contact d'assise (21754), dans lequel le premier contact d'assise (21752) est en communication électrique avec le second contact d'assise (21754) ; et
un second loquet (21730) accouplé à l'ensemble poignée (29000), dans lequel le second loquet (21730) est conçu pour mettre en prise le premier loquet (21716) afin de coupler l'ensemble adaptateur (29002) à l'ensemble poignée (29000), et dans lequel le second loquet (21730) comprend :
un premier doigt (21734) comprenant un premier contact de doigt (21762), dans lequel le premier contact de doigt (21762) est configuré pour se coupler électriquement au premier contact d'assise (21752) sur la base de du premier doigt (21734) entrant en prise avec la première plate-forme d'assise (21718) ; et
un second doigt (21736) comprenant un second contact de doigt (21764), dans lequel le second contact de doigt (21764) est configuré pour se coupler électriquement au second contact d'assise (21754) sur la base de du second doigt (21736) entrant en prise avec la seconde plate-forme d'assise (21722).

9. Système chirurgical selon la revendication 6 ou 7, dans lequel le second ensemble détecteur comprend :
un premier loquet (21716) accouplé à l'ensemble adaptateur (29002), dans lequel le premier loquet (21716) comprend :
une première plate-forme d'assise (21718) ; et
une seconde plate-forme d'assise (21722) ; et
un second loquet (21730) accouplé à l'ensemble poignée (29000), dans lequel le second loquet (21730) est conçu pour mettre en prise le premier loquet (21716) afin de coupler l'ensemble adaptateur (29002) à l'ensemble poignée (29000), et dans lequel le second loquet (21730) comprend :
un premier doigt (21734) comprenant un premier interrupteur marche-arrêt (21770), dans lequel le premier interrupteur marche-arrêt (21770) est configuré pour s'actionner lorsque le premier interrupteur marche-arrêt (21770) entre en prise avec la première plate-forme d'assise (21718) ; et
un second doigt (21736) comprenant un second interrupteur marche-arrêt (21772), dans lequel le second interrupteur marche-arrêt (21772) est configuré pour s'actionner lorsque le second interrupteur marche-arrêt (21772) entre en prise avec la seconde plate-forme d'assise (21722).

10. Système chirurgical selon la revendication 6, dans lequel le second ensemble détecteur comprend :
un troisième contact (21020a) disposé sur l'ensemble poignée (29000) ;
un quatrième contact (21020b) disposé sur l'ensemble poignée (29000) ;
un premier arbre pouvant être enfoncé (21016a) s'étendant à partir de l'ensemble adaptateur (29002), dans lequel le premier arbre pouvant être enfoncé (21016a) est conçu pour mettre en prise le troisième contact (21020a) ; et
un second arbre pouvant être enfoncé (21016b) s'étendant à partir de l'ensemble adaptateur (29002), dans lequel le second arbre pouvant être enfoncé (21016b) est conçu pour mettre en prise le quatrième contact (21020b).
